# EUROPEAN PATENT SPECIFICATION

(11) **EP 3 506 270 B1**
(45) Date of publication and mention of the grant of the patent: **08.05.2024**
(21) Application number: 18275226.1
(22) Date of filing: 28.12.2018
(51) Int. Cl.: G16H 10/60, G16H 15/00

(54) **COMMUNICATION OF DATA WHERE A SURGICAL NETWORK IS USING CONTEXT OF THE DATA AND REQUIREMENTS OF A RECEIVING SYSTEM / USER TO INFLUENCE INCLUSION OR LINKAGE OF DATA AND METADATA TO ESTABLISH CONTINUITY**
KOMMUNIKATION VON DATEN, WENN EIN CHIRURGISCHES NETZWERK DEN KONTEXT DER DATEN UND ANFORDERUNGEN EINES EMPFANGSSYSTEMS/BENUTZERS NUTZT, UM DIE EINBEZIEHUNG ODER VERKNÜPFUNG VON DATEN UND METADATEN ZU BEEINFLUSSEN, UM KONTINUITÄT HERZUSTELLEN
COMMUNICATION DE DONNÉES LORSQU'UN RÉSEAU CHIRURGICAL UTILISE LE CONTEXTE DES DONNÉES ET LES EXIGENCES D'UN SYSTÈME/UTILISATEUR RÉCEPTEUR POUR INFLUENCER L'INCLUSION OU LE LIEN DE DONNÉES ET DE MÉTADONNÉES POUR ÉTABLIR LA CONTINUITÉ

(30) Priority: 28.12.2017 US 201762611341 P; 28.12.2017 US 201762611340 P; 28.12.2017 US 201762611339 P; 08.03.2018 US 201862640415 P; 08.03.2018 US 201862640417 P; 30.03.2018 US 201862650898 P; 30.03.2018 US 201862650882 P; 30.03.2018 US 201862650877 P; 30.03.2018 US 201862650887 P; 19.04.2018 US 201862659900 P; 30.06.2018 US 201862692768 P; 30.06.2018 US 201862692748 P; 30.06.2018 US 201862692747 P; 10.09.2018 US 201862729191 P; 06.11.2018 US 201816182278
(43) Date of publication of application: 03.07.2019
(73) Proprietor: Ethicon LLC, Guaynabo 00969 (PR)
(72) Inventor: SHELTON, IV Frederick E., Cincinnati, OH Ohio 45242 (US); HARRIS, Jason L., Cincinnati, OH Ohio 45242 (US)
(74) Representative: Carpmaels & Ransford LLP

(56) References cited:
- EP-A1- 1 769 771
- WO-A1-2017/083768
- US-A1- 2016 253 472

## Description

### BACKGROUND

The present disclosure relates to various surgical systems. Surgical procedures are typically performed in surgical operating theaters or rooms in a healthcare facility such as, for example, a hospital. A sterile field is typically created around the patient. The sterile field may include the scrubbed team members, who are properly attired, and all furniture and fixtures in the area. Various surgical devices and systems are utilized in performance of a surgical procedure.
WO2017/083768A discloses controlling a robot-assisted surgical system to gather and assess surgery-performance data to automatically assist or advise surgical personnel wherein these data are stored in a database. This document fails to determine a surgical context based on the perioperative data and define a relationship between two databases based on the surgical context.

### SUMMARY

The present invention provides a computer system configured to be communicably coupled to a surgical device and a database system as recited in claim 1. Optional features are recited in the dependent claims.

The present invention also provides a computer-implemented method for sharing data between a computer system and a database system as recited in claim 2. Optional features are recited in the dependent claims.

The present invention also provides a computer system configured to be communicably coupled to a plurality of surgical devices and a database as recited in claim 6. Optional features are recited in the dependent claims.

### FIGURES

The various aspects described herein, both as to organization and methods of operation, together with further objects and advantages thereof, may best be understood by reference to the following description, taken in conjunction with the accompanying drawings as follows.
FIG. 1 is a block diagram of a computer-implemented interactive surgical system, in accordance with at least one aspect of the present disclosure.
FIG. 2 is a surgical system being used to perform a surgical procedure in an operating room, in accordance with at least one aspect of the present disclosure.
FIG. 3 is a surgical hub paired with a visualization system, a robotic system, and an intelligent instrument, in accordance with at least one aspect of the present disclosure.
FIG. 4 is a partial perspective view of a surgical hub enclosure, and of a combo generator module slidably receivable in a drawer of the surgical hub enclosure, in accordance with at least one aspect of the present disclosure.
FIG. 5 is a perspective view of a combo generator module with bipolar, ultrasonic, and monopolar contacts and a smoke evacuation component, in accordance with at least one aspect of the present disclosure.
FIG. 6 illustrates individual power bus attachments for a plurality of lateral docking ports of a lateral modular housing configured to receive a plurality of modules, in accordance with at least one aspect of the present disclosure.
FIG. 7 illustrates a vertical modular housing configured to receive a plurality of modules, in accordance with at least one aspect of the present disclosure.
FIG. 8 illustrates a surgical data network comprising a modular communication hub configured to connect modular devices located in one or more operating theaters of a healthcare facility, or any room in a healthcare facility specially equipped for surgical operations, to the cloud, in accordance with at least one aspect of the present disclosure.
FIG. 9 illustrates a computer-implemented interactive surgical system, in accordance with at least one aspect of the present disclosure.
FIG. 10 illustrates a surgical hub comprising a plurality of modules coupled to the modular control tower, in accordance with at least one aspect of the present disclosure.
FIG. 11 illustrates one aspect of a Universal Serial Bus (USB) network hub device, in accordance with at least one aspect of the present disclosure.
FIG. 12 is a block diagram of a cloud computing system comprising a plurality of smart surgical instruments coupled to surgical hubs that may connect to the cloud component of the cloud computing system, in accordance with at least one aspect of the present disclosure.
FIG. 13 is a functional module architecture of a cloud computing system, in accordance with at least one aspect of the present disclosure.
FIG. 14 illustrates a diagram of a situationally aware surgical system, in accordance with at least one aspect of the present disclosure.
FIG. 15 is a timeline depicting situational awareness of a surgical hub, in accordance with at least one aspect of the present disclosure.
FIG. 16 is a diagram of a database system illustrating data interoperability between interrelated databases, in accordance with at least one aspect of the present disclosure.
FIG. 17 is a diagram of a database system illustrating data fluidity between interrelated databases, in accordance with at least one aspect of the present disclosure.
FIG. 18 is a logic flow diagram of a process for sharing data between databases, in accordance with at least one aspect of the present disclosure.
FIG. 19 is a diagram of a database system where particular data is shared between surgical hub, electronic health record (EHR), and hospital administration databases, in accordance with at least one aspect of the present disclosure.
FIG. 20 is a diagram of a database system where particular data is shared between EHR and hospital administration databases, in accordance with at least one aspect of the present disclosure.
FIG. 21 is a diagram illustrating a security and authorization system for a medical facility database system, in accordance with at least one aspect of the present disclosure.

### DESCRIPTION

Before explaining various aspects of surgical devices and generators in detail, it should be noted that the illustrative examples are not limited in application or use to the details of construction and arrangement of parts illustrated in the accompanying drawings and description. The illustrative examples may be implemented or incorporated in other aspects, variations and modifications, and may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following-described aspects, expressions of aspects, and/or examples, can be combined with any one or more of the other following-described aspects, expressions of aspects and/or examples.

### Surgical Hubs

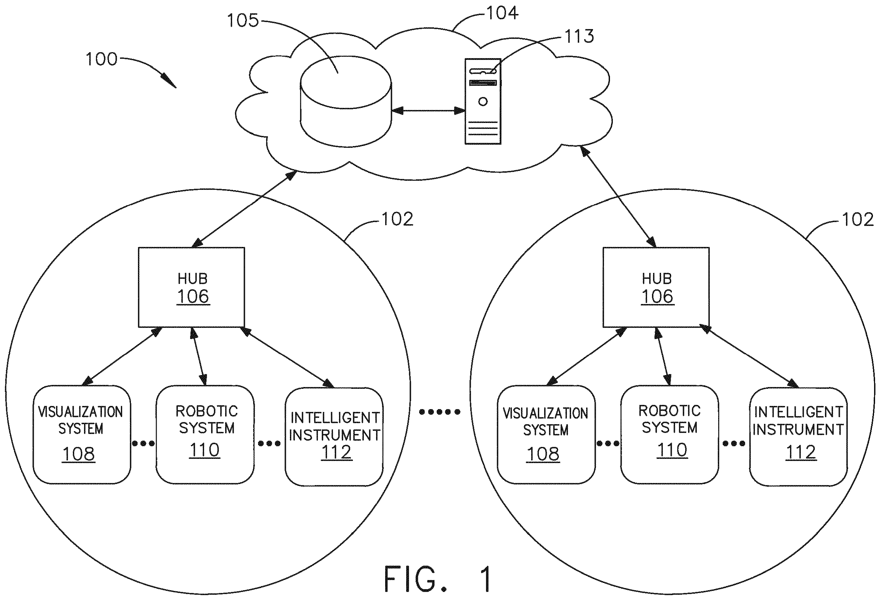

Referring to FIG. 1, a computer-implemented interactive surgical system 100 includes one or more surgical systems 102 and a cloud-based system (e.g., the cloud 104 that may include a remote server 113 coupled to a storage device 105). Each surgical system 102 includes at least one surgical hub 106 in communication with the cloud 104 that may include a remote server 113. In one example, as illustrated in FIG. 1, the surgical system 102 includes a visualization system 108, a robotic system 110, and a handheld intelligent surgical instrument 112, which are configured to communicate with one another and/or the hub 106. In some aspects, a surgical system 102 may include an M number of hubs 106, an N number of visualization systems 108, an O number of robotic systems 110, and a P number of handheld intelligent surgical instruments 112, where M, N, O, and P are integers greater than or equal to one.

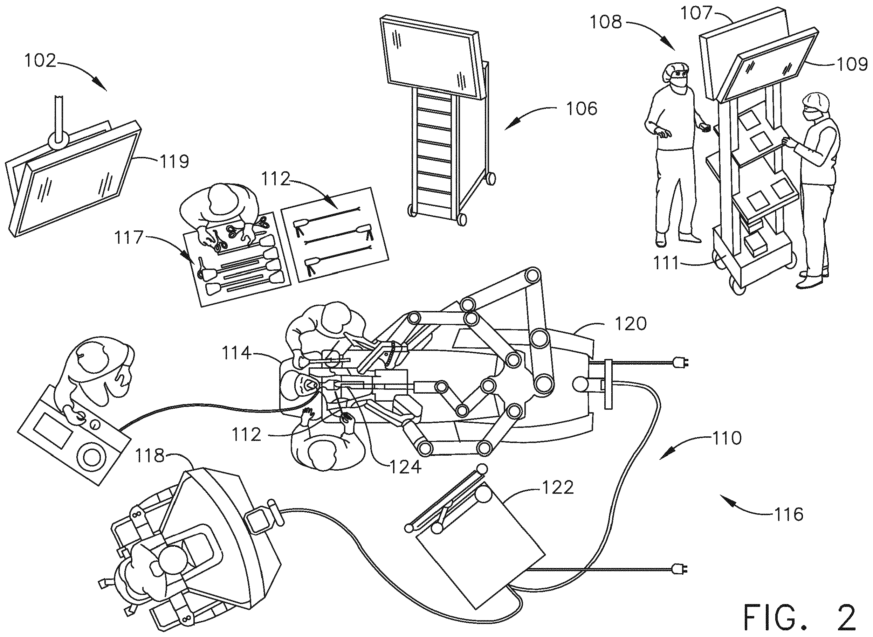

FIG. 2 depicts an example of a surgical system 102 being used to perform a surgical procedure on a patient who is lying down on an operating table 114 in a surgical operating room 116. A robotic system 110 is used in the surgical procedure as a part of the surgical system 102. The robotic system 110 includes a surgeon's console 118, a patient side cart 120 (surgical robot), and a surgical robotic hub 122. The patient side cart 120 can manipulate at least one removably coupled surgical tool 117 through a minimally invasive incision in the body of the patient while the surgeon views the surgical site through the surgeon's console 118. An image of the surgical site can be obtained by a medical imaging device 124, which can be manipulated by the patient side cart 120 to orient the imaging device 124. The robotic hub 122 can be used to process the images of the surgical site for subsequent display to the surgeon through the surgeon's console 118.

Other types of robotic systems can be readily adapted for use with the surgical system 102. Various examples of robotic systems and surgical tools that are suitable for use with the present disclosure are described in U.S. Provisional Patent Application Serial No. 62/611,339, titled ROBOT ASSISTED SURGICAL PLATFORM, filed December 28, 2017.

Various examples of cloud-based analytics that are performed by the cloud 104, and are suitable for use with the present disclosure, are described in U.S. Provisional Patent Application Serial No. 62/611,340, titled CLOUD-BASED MEDICAL ANALYTICS, filed December 28, 2017.

In various aspects, the imaging device 124 includes at least one image sensor and one or more optical components. Suitable image sensors include, but are not limited to, Charge-Coupled Device (CCD) sensors and Complementary Metal-Oxide Semiconductor (CMOS) sensors.

The optical components of the imaging device 124 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The one or more illumination sources may be configured to radiate electromagnetic energy in the visible spectrum as well as the invisible spectrum. The visible spectrum, sometimes referred to as the optical spectrum or luminous spectrum, is that portion of the electromagnetic spectrum that is visible to (i.e., can be detected by) the human eye and may be referred to as visible light or simply light. A typical human eye will respond to wavelengths in air that are from about 380 nm to about 750 nm.

The invisible spectrum (i.e., the non-luminous spectrum) is that portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 124 is configured for use in a minimally invasive procedure. Examples of imaging devices suitable for use with the present disclosure include, but not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

In one aspect, the imaging device employs multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information the human eye fails to capture with its receptors for red, green, and blue. The use of multi-spectral imaging is described in greater detail under the heading "Advanced Imaging Acquisition Module" in U.S. Provisional Patent Application Serial No. 62/611,341, titled INTERACTIVE SURGICAL PLATFORM, filed December 28, 2017. Multi-spectrum monitoring can be a useful tool in relocating a surgical field after a surgical task is completed to perform one or more of the previously described tests on the treated tissue.

It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device 124 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

In various aspects, the visualization system 108 includes one or more imaging sensors, one or more image-processing units, one or more storage arrays, and one or more displays that are strategically arranged with respect to the sterile field, as illustrated in FIG. 2. In one aspect, the visualization system 108 includes an interface for HL7, PACS, and EMR. Various components of the visualization system 108 are described under the heading "Advanced Imaging Acquisition Module" in U.S. Provisional Patent Application Serial No. 62/611,341, titled INTERACTIVE SURGICAL PLATFORM, filed December 28, 2017.

As illustrated in FIG. 2, a primary display 119 is positioned in the sterile field to be visible to an operator at the operating table 114. In addition, a visualization tower 111 is positioned outside the sterile field. The visualization tower 111 includes a first non-sterile display 107 and a second non-sterile display 109, which face away from each other. The visualization system 108, guided by the hub 106, is configured to utilize the displays 107, 109, and 119 to coordinate information flow to operators inside and outside the sterile field. For example, the hub 106 may cause the visualization system 108 to display a snapshot of a surgical site, as recorded by an imaging device 124, on a non-sterile display 107 or 109, while maintaining a live feed of the surgical site on the primary display 119. The snapshot on the non-sterile display 107 or 109 can permit a non-sterile operator to perform a diagnostic step relevant to the surgical procedure, for example.

In one aspect, the hub 106 is also configured to route a diagnostic input or feedback entered by a non-sterile operator at the visualization tower 111 to the primary display 119 within the sterile field, where it can be viewed by a sterile operator at the operating table. In one example, the input can be in the form of a modification to the snapshot displayed on the non-sterile display 107 or 109, which can be routed to the primary display 119 by the hub 106.

Referring to FIG. 2, a surgical instrument 112 is being used in the surgical procedure as part of the surgical system 102. The hub 106 is also configured to coordinate information flow to a display of the surgical instrument 112. For example, coordinate information flow is further described in U.S. Provisional Patent Application Serial No. 62/611,341, titled INTERACTIVE SURGICAL PLATFORM, filed December 28, 2017. A diagnostic input or feedback entered by a non-sterile operator at the visualization tower 111 can be routed by the hub 106 to the surgical instrument display 115 within the sterile field, where it can be viewed by the operator of the surgical instrument 112. Example surgical instruments that are suitable for use with the surgical system 102 are described under the heading "Surgical instrument Hardware" in U.S. Provisional Patent Application Serial No. 62/611,341, titled INTERACTIVE SURGICAL PLATFORM, filed December 28, 2017, for example.

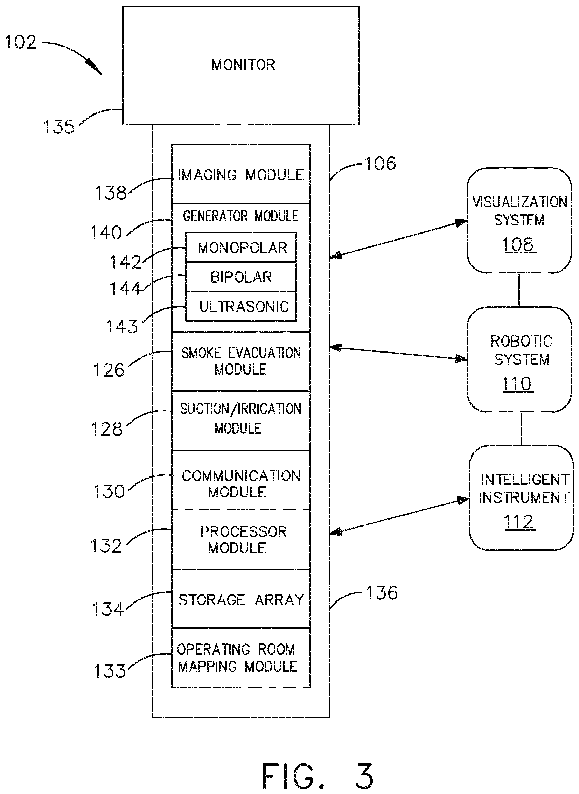

Referring now to FIG. 3, a hub 106 is depicted in communication with a visualization system 108, a robotic system 110, and a handheld intelligent surgical instrument 112. The hub 106 includes a hub display 135, an imaging module 138, a generator module 140 (which can include a monopolar generator 142, a bipolar generator 144, and/or an ultrasonic generator 143), a communication module 130, a processor module 132, and a storage array 134. In certain aspects, as illustrated in FIG. 3, the hub 106 further includes a smoke evacuation module 126, a suction/irrigation module 128, and/or an OR mapping module 133.

During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure 136 offers a unified environment for managing the power, data, and fluid lines, which reduces the frequency of entanglement between such lines.

Aspects of the present disclosure present a surgical hub for use in a surgical procedure that involves energy application to tissue at a surgical site. The surgical hub includes a hub enclosure and a combo generator module slidably receivable in a docking station of the hub enclosure. The docking station includes data and power contacts. The combo generator module includes two or more of an ultrasonic energy generator component, a bipolar RF energy generator component, and a monopolar RF energy generator component that are housed in a single unit. In one aspect, the combo generator module also includes a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component.

In one aspect, the fluid line is a first fluid line and a second fluid line extends from the remote surgical site to a suction and irrigation module slidably received in the hub enclosure. In one aspect, the hub enclosure comprises a fluid interface.

Certain surgical procedures may require the application of more than one energy type to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 136 is configured to accommodate different generators, and facilitate an interactive communication therebetween. One of the advantages of the hub modular enclosure 136 is enabling the quick removal and/or replacement of various modules.

Aspects of the present disclosure present a modular surgical enclosure for use in a surgical procedure that involves energy application to tissue. The modular surgical enclosure includes a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts,

Further to the above, the modular surgical enclosure also includes a second energy-generator module configured to generate a second energy, different than the first energy, for application to the tissue, and a second docking station comprising a second docking port that includes second data and power contacts, wherein the second energy-generator module is slidably movable into an electrical engagement with the power and data contacts, and wherein the second energy-generator module is slidably movable out of the electrical engagement with the second power and data contacts.

In addition, the modular surgical enclosure also includes a communication bus between the first docking port and the second docking port, configured to facilitate communication between the first energy-generator module and the second energy-generator module.

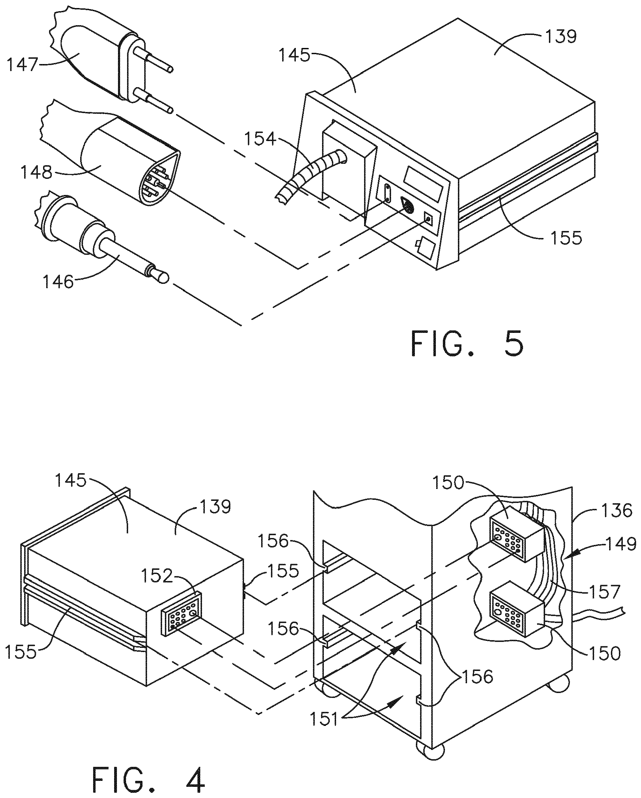

Referring to FIGS. 3-7, aspects of the present disclosure are presented for a hub modular enclosure 136 that allows the modular integration of a generator module 140, a smoke evacuation module 126, and a suction/irrigation module 128. The hub modular enclosure 136 further facilitates interactive communication between the modules 140, 126, 128. As illustrated in FIG. 5, the generator module 140 can be a generator module with integrated monopolar, bipolar, and ultrasonic components supported in a single housing unit 139 slidably insertable into the hub modular enclosure 136. As illustrated in FIG. 5, the generator module 140 can be configured to connect to a monopolar device 146, a bipolar device 147, and an ultrasonic device 148. Alternatively, the generator module 140 may comprise a series of monopolar, bipolar, and/or ultrasonic generator modules that interact through the hub modular enclosure 136. The hub modular enclosure 136 can be configured to facilitate the insertion of multiple generators and interactive communication between the generators docked into the hub modular enclosure 136 so that the generators would act as a single generator.

In one aspect, the hub modular enclosure 136 comprises a modular power and communication backplane 149 with external and wireless communication headers to enable the removable attachment of the modules 140, 126, 128 and interactive communication therebetween.

In one aspect, the hub modular enclosure 136 includes docking stations, or drawers, 151, herein also referred to as drawers, which are configured to slidably receive the modules 140, 126, 128. FIG. 4 illustrates a partial perspective view of a surgical hub enclosure 136, and a combo generator module 145 slidably receivable in a docking station 151 of the surgical hub enclosure 136. A docking port 152 with power and data contacts on a rear side of the combo generator module 145 is configured to engage a corresponding docking port 150 with power and data contacts of a corresponding docking station 151 of the hub modular enclosure 136 as the combo generator module 145 is slid into position within the corresponding docking station 151 of the hub module enclosure 136. In one aspect, the combo generator module 145 includes a bipolar, ultrasonic, and monopolar module and a smoke evacuation module integrated together into a single housing unit 139, as illustrated in FIG. 5.

In various aspects, the smoke evacuation module 126 includes a fluid line 154 that conveys captured/collected smoke and/or fluid away from a surgical site and to, for example, the smoke evacuation module 126. Vacuum suction originating from the smoke evacuation module 126 can draw the smoke into an opening of a utility conduit at the surgical site. The utility conduit, coupled to the fluid line, can be in the form of a flexible tube terminating at the smoke evacuation module 126. The utility conduit and the fluid line define a fluid path extending toward the smoke evacuation module 126 that is received in the hub enclosure 136.

In various aspects, the suction/irrigation module 128 is coupled to a surgical tool comprising an aspiration fluid line and a suction fluid line. In one example, the aspiration and suction fluid lines are in the form of flexible tubes extending from the surgical site toward the suction/irrigation module 128. One or more drive systems can be configured to cause irrigation and aspiration of fluids to and from the surgical site.

In one aspect, the surgical tool includes a shaft having an end effector at a distal end thereof and at least one energy treatment associated with the end effector, an aspiration tube, and an irrigation tube. The aspiration tube can have an inlet port at a distal end thereof and the aspiration tube extends through the shaft. Similarly, an irrigation tube can extend through the shaft and can have an inlet port in proximity to the energy deliver implement. The energy deliver implement is configured to deliver ultrasonic and/or RF energy to the surgical site and is coupled to the generator module 140 by a cable extending initially through the shaft.

The irrigation tube can be in fluid communication with a fluid source, and the aspiration tube can be in fluid communication with a vacuum source. The fluid source and/or the vacuum source can be housed in the suction/irrigation module 128. In one example, the fluid source and/or the vacuum source can be housed in the hub enclosure 136 separately from the suction/irrigation module 128. In such example, a fluid interface can be configured to connect the suction/irrigation module 128 to the fluid source and/or the vacuum source.

In one aspect, the modules 140, 126, 128 and/or their corresponding docking stations on the hub modular enclosure 136 may include alignment features that are configured to align the docking ports of the modules into engagement with their counterparts in the docking stations of the hub modular enclosure 136. For example, as illustrated in FIG. 4, the combo generator module 145 includes side brackets 155 that are configured to slidably engage with corresponding brackets 156 of the corresponding docking station 151 of the hub modular enclosure 136. The brackets cooperate to guide the docking port contacts of the combo generator module 145 into an electrical engagement with the docking port contacts of the hub modular enclosure 136.

In some aspects, the drawers 151 of the hub modular enclosure 136 are the same, or substantially the same size, and the modules are adjusted in size to be received in the drawers 151. For example, the side brackets 155 and/or 156 can be larger or smaller depending on the size of the module. In other aspects, the drawers 151 are different in size and are each designed to accommodate a particular module.

Furthermore, the contacts of a particular module can be keyed for engagement with the contacts of a particular drawer to avoid inserting a module into a drawer with mismatching contacts.

As illustrated in FIG. 4, the docking port 150 of one drawer 151 can be coupled to the docking port 150 of another drawer 151 through a communications link 157 to facilitate an interactive communication between the modules housed in the hub modular enclosure 136. The docking ports 150 of the hub modular enclosure 136 may alternatively, or additionally, facilitate a wireless interactive communication between the modules housed in the hub modular enclosure 136. Any suitable wireless communication can be employed, such as for example Air Titan-Bluetooth.

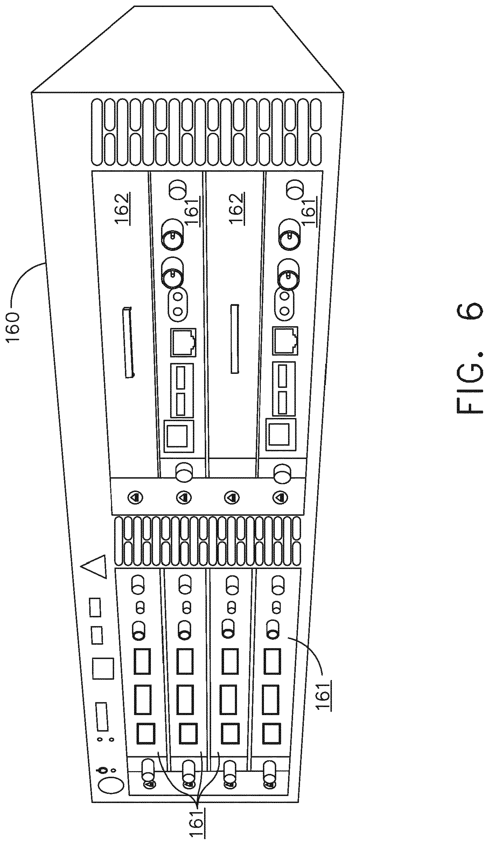

FIG. 6 illustrates individual power bus attachments for a plurality of lateral docking ports of a lateral modular housing 160 configured to receive a plurality of modules of a surgical hub 206. The lateral modular housing 160 is configured to laterally receive and interconnect the modules 161. The modules 161 are slidably inserted into docking stations 162 of lateral modular housing 160, which includes a backplane for interconnecting the modules 161. As illustrated in FIG. 6, the modules 161 are arranged laterally in the lateral modular housing 160. Alternatively, the modules 161 may be arranged vertically in a lateral modular housing.

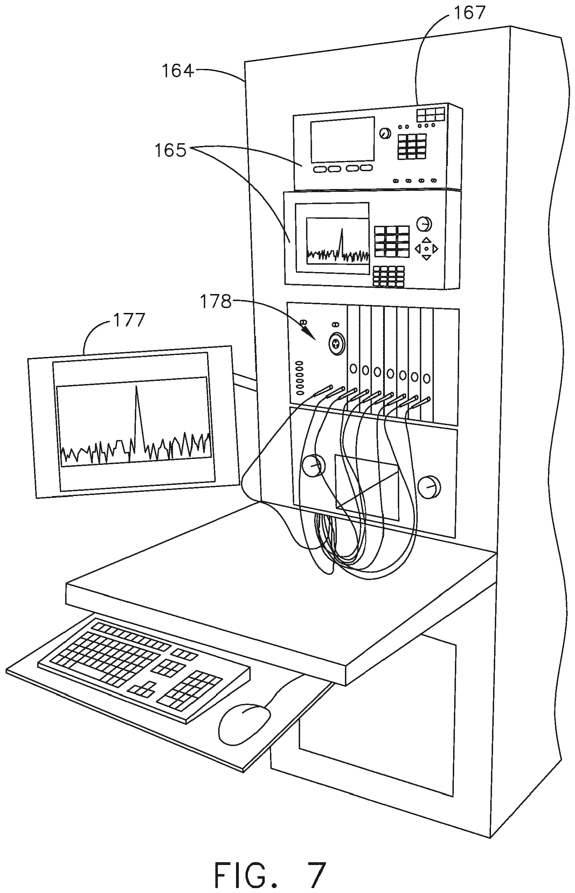

FIG. 7 illustrates a vertical modular housing 164 configured to receive a plurality of modules 165 of the surgical hub 106. The modules 165 are slidably inserted into docking stations, or drawers, 167 of vertical modular housing 164, which includes a backplane for interconnecting the modules 165. Although the drawers 167 of the vertical modular housing 164 are arranged vertically, in certain instances, a vertical modular housing 164 may include drawers that are arranged laterally. Furthermore, the modules 165 may interact with one another through the docking ports of the vertical modular housing 164. In the example of FIG. 7, a display 177 is provided for displaying data relevant to the operation of the modules 165. In addition, the vertical modular housing 164 includes a master module 178 housing a plurality of sub-modules that are slidably received in the master module 178.

In various aspects, the imaging module 138 comprises an integrated video processor and a modular light source and is adapted for use with various imaging devices. In one aspect, the imaging device is comprised of a modular housing that can be assembled with a light source module and a camera module. The housing can be a disposable housing. In at least one example, the disposable housing is removably coupled to a reusable controller, a light source module, and a camera module. The light source module and/or the camera module can be selectively chosen depending on the type of surgical procedure. In one aspect, the camera module comprises a CCD sensor. In another aspect, the camera module comprises a CMOS sensor. In another aspect, the camera module is configured for scanned beam imaging. Likewise, the light source module can be configured to deliver a white light or a different light, depending on the surgical procedure.

During a surgical procedure, removing a surgical device from the surgical field and replacing it with another surgical device that includes a different camera or a different light source can be inefficient. Temporarily losing sight of the surgical field may lead to undesirable consequences. The module imaging device of the present disclosure is configured to permit the replacement of a light source module or a camera module midstream during a surgical procedure, without having to remove the imaging device from the surgical field.

In one aspect, the imaging device comprises a tubular housing that includes a plurality of channels. A first channel is configured to slidably receive the camera module, which can be configured for a snap-fit engagement with the first channel. A second channel is configured to slidably receive the light source module, which can be configured for a snap-fit engagement with the second channel. In another example, the camera module and/or the light source module can be rotated into a final position within their respective channels. A threaded engagement can be employed in lieu of the snap-fit engagement.

In various examples, multiple imaging devices are placed at different positions in the surgical field to provide multiple views. The imaging module 138 can be configured to switch between the imaging devices to provide an optimal view. In various aspects, the imaging module 138 can be configured to integrate the images from the different imaging device.

Various image processors and imaging devices suitable for use with the present disclosure are described in U.S. Patent No. 7,995,045, titled COMBINED SBI AND CONVENTIONAL IMAGE PROCESSOR, which issued on August 9, 2011. In addition, U.S. Patent No. 7,982,776, titled SBI MOTION ARTIFACT REMOVAL APPARATUS AND METHOD, which issued on July 19, 2011 describes various systems for removing motion artifacts from image data. Such systems can be integrated with the imaging module 138. Furthermore, U.S. Patent Application Publication No. 2011/0306840, titled CONTROLLABLE MAGNETIC SOURCE TO FIXTURE INTRACORPOREAL APPARATUS, which published on December 15, 2011, and U.S. Patent Application Publication No. 2014/0243597, titled SYSTEM FOR PERFORMING A MINIMALLY INVASIVE SURGICAL PROCEDURE, which published on August 28, 2014.

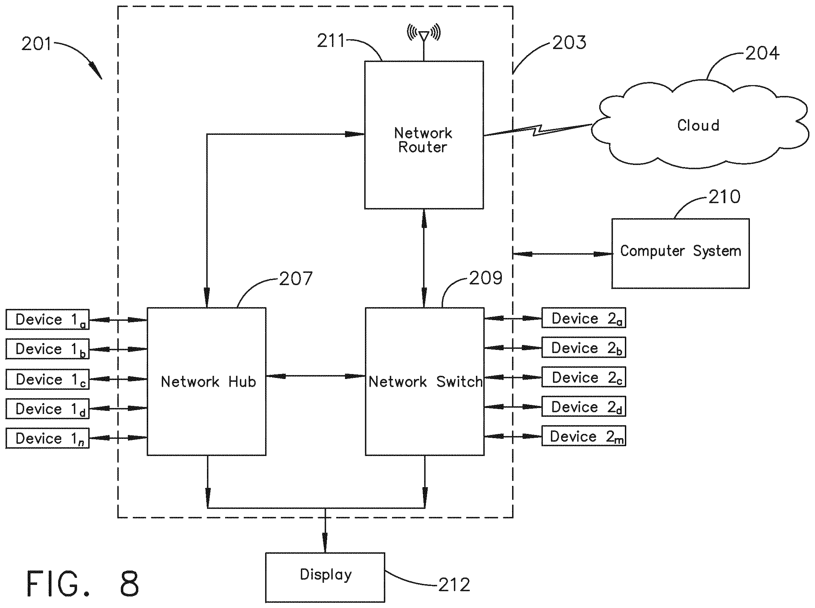

FIG. 8 illustrates a surgical data network 201 comprising a modular communication hub 203 configured to connect modular devices located in one or more operating theaters of a healthcare facility, or any room in a healthcare facility specially equipped for surgical operations, to a cloud-based system (e.g., the cloud 204 that may include a remote server 213 coupled to a storage device 205). In one aspect, the modular communication hub 203 comprises a network hub 207 and/or a network switch 209 in communication with a network router. The modular communication hub 203 also can be coupled to a local computer system 210 to provide local computer processing and data manipulation. The surgical data network 201 may be configured as passive, intelligent, or switching. A passive surgical data network serves as a conduit for the data, enabling it to go from one device (or segment) to another and to the cloud computing resources. An intelligent surgical data network includes additional features to enable the traffic passing through the surgical data network to be monitored and to configure each port in the network hub 207 or network switch 209. An intelligent surgical data network may be referred to as a manageable hub or switch. A switching hub reads the destination address of each packet and then forwards the packet to the correct port.

Modular devices 1a-1n located in the operating theater may be coupled to the modular communication hub 203. The network hub 207 and/or the network switch 209 may be coupled to a network router 211 to connect the devices 1a-1n to the cloud 204 or the local computer system 210. Data associated with the devices 1a-1n may be transferred to cloud-based computers via the router for remote data processing and manipulation. Data associated with the devices 1a-1n may also be transferred to the local computer system 210 for local data processing and manipulation. Modular devices 2a-2m located in the same operating theater also may be coupled to a network switch 209. The network switch 209 may be coupled to the network hub 207 and/or the network router 211 to connect to the devices 2a-2m to the cloud 204. Data associated with the devices 2a-2n may be transferred to the cloud 204 via the network router 211 for data processing and manipulation. Data associated with the devices 2a-2m may also be transferred to the local computer system 210 for local data processing and manipulation.

It will be appreciated that the surgical data network 201 may be expanded by interconnecting multiple network hubs 207 and/or multiple network switches 209 with multiple network routers 211. The modular communication hub 203 may be contained in a modular control tower configured to receive multiple devices 1a-1n/2a-2m. The local computer system 210 also may be contained in a modular control tower. The modular communication hub 203 is connected to a display 212 to display images obtained by some of the devices 1a-1n/2a-2m, for example during surgical procedures. In various aspects, the devices 1a-1n/2a-2m may include, for example, various modules such as an imaging module 138 coupled to an endoscope, a generator module 140 coupled to an energy-based surgical device, a smoke evacuation module 126, a suction/irrigation module 128, a communication module 130, a processor module 132, a storage array 134, a surgical device coupled to a display, and/or a non-contact sensor module, among other modular devices that may be connected to the modular communication hub 203 of the surgical data network 201.

In one aspect, the surgical data network 201 may comprise a combination of network hub(s), network switch(es), and network router(s) connecting the devices 1a-1n/2a-2m to the cloud. Any one of or all of the devices 1a-1n/2a-2m coupled to the network hub or network switch may collect data in real time and transfer the data to cloud computers for data processing and manipulation. It will be appreciated that cloud computing relies on sharing computing resources rather than having local servers or personal devices to handle software applications. The word "cloud" may be used as a metaphor for "the Internet," although the term is not limited as such. Accordingly, the term "cloud computing" may be used herein to refer to "a type of Internet-based computing," where different services-such as servers, storage, and applications-are delivered to the modular communication hub 203 and/or computer system 210 located in the surgical theater (e.g., a fixed, mobile, temporary, or field operating room or space) and to devices connected to the modular communication hub 203 and/or computer system 210 through the Internet. The cloud infrastructure may be maintained by a cloud service provider. In this context, the cloud service provider may be the entity that coordinates the usage and control of the devices 1a-1n/2a-2m located in one or more operating theaters. The cloud computing services can perform a large number of calculations based on the data gathered by smart surgical instruments, robots, and other computerized devices located in the operating theater. The hub hardware enables multiple devices or connections to be connected to a computer that communicates with the cloud computing resources and storage.

Applying cloud computer data processing techniques on the data collected by the devices 1a-1n/2a-2m, the surgical data network provides improved surgical outcomes, reduced costs, and improved patient satisfaction. At least some of the devices 1a-1n/2a-2m may be employed to view tissue states to assess leaks or perfusion of sealed tissue after a tissue sealing and cutting procedure. At least some of the devices 1a-1n/2a-2m may be employed to identify pathology, such as the effects of diseases, using the cloud-based computing to examine data including images of samples of body tissue for diagnostic purposes. This includes localization and margin confirmation of tissue and phenotypes. At least some of the devices 1a-1n/2a-2m may be employed to identify anatomical structures of the body using a variety of sensors integrated with imaging devices and techniques such as overlaying images captured by multiple imaging devices. The data gathered by the devices 1a-1n/2a-2m, including image data, may be transferred to the cloud 204 or the local computer system 210 or both for data processing and manipulation including image processing and manipulation. The data may be analyzed to improve surgical procedure outcomes by determining if further treatment, such as the application of endoscopic intervention, emerging technologies, a targeted radiation, targeted intervention, and precise robotics to tissue-specific sites and conditions, may be pursued. Such data analysis may further employ outcome analytics processing, and using standardized approaches may provide beneficial feedback to either confirm surgical treatments and the behavior of the surgeon or suggest modifications to surgical treatments and the behavior of the surgeon.

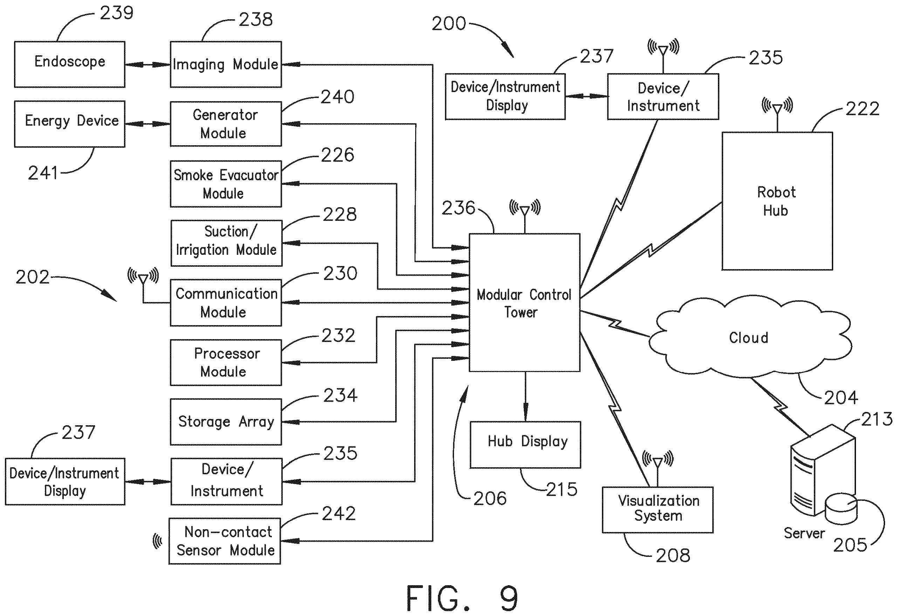

In one implementation, the operating theater devices 1a-1n may be connected to the modular communication hub 203 over a wired channel or a wireless channel depending on the configuration of the devices 1a-1n to a network hub. The network hub 207 may be implemented, in one aspect, as a local network broadcast device that works on the physical layer of the Open System Interconnection (OSI) model. The network hub provides connectivity to the devices 1a-1n located in the same operating theater network. The network hub 207 collects data in the form of packets and sends them to the router in half duplex mode. The network hub 207 does not store any media access control/Internet Protocol (MAC/IP) to transfer the device data. Only one of the devices 1a-1n can send data at a time through the network hub 207. The network hub 207 has no routing tables or intelligence regarding where to send information and broadcasts all network data across each connection and to a remote server 213 (FIG. 9) over the cloud 204. The network hub 207 can detect basic network errors such as collisions, but having all information broadcast to multiple ports can be a security risk and cause bottlenecks.

In another implementation, the operating theater devices 2a-2m may be connected to a network switch 209 over a wired channel or a wireless channel. The network switch 209 works in the data link layer of the OSI model. The network switch 209 is a multicast device for connecting the devices 2a-2m located in the same operating theater to the network. The network switch 209 sends data in the form of frames to the network router 211 and works in full duplex mode. Multiple devices 2a-2m can send data at the same time through the network switch 209. The network switch 209 stores and uses MAC addresses of the devices 2a-2m to transfer data.

The network hub 207 and/or the network switch 209 are coupled to the network router 211 for connection to the cloud 204. The network router 211 works in the network layer of the OSI model. The network router 211 creates a route for transmitting data packets received from the network hub 207 and/or network switch 211 to cloud-based computer resources for further processing and manipulation of the data collected by any one of or all the devices 1a-1n/2a-2m. The network router 211 may be employed to connect two or more different networks located in different locations, such as, for example, different operating theaters of the same healthcare facility or different networks located in different operating theaters of different healthcare facilities. The network router 211 sends data in the form of packets to the cloud 204 and works in full duplex mode. Multiple devices can send data at the same time. The network router 211 uses IP addresses to transfer data.

In one example, the network hub 207 may be implemented as a USB hub, which allows multiple USB devices to be connected to a host computer. The USB hub may expand a single USB port into several tiers so that there are more ports available to connect devices to the host system computer. The network hub 207 may include wired or wireless capabilities to receive information over a wired channel or a wireless channel. In one aspect, a wireless USB short-range, high-bandwidth wireless radio communication protocol may be employed for communication between the devices 1a-1n and devices 2a-2m located in the operating theater.

In other examples, the operating theater devices 1a-1n/2a-2m may communicate to the modular communication hub 203 via Bluetooth wireless technology standard for exchanging data over short distances (using short-wavelength UHF radio waves in the ISM band from 2.4 to 2.485 GHz) from fixed and mobile devices and building personal area networks (PANs). In other aspects, the operating theater devices 1a-1n/2a-2m may communicate to the modular communication hub 203 via a number of wireless or wired communication standards or protocols, including but not limited to Wi-Fi (IEEE 802.11 family), WiMAX (IEEE 802.16 family), IEEE 802.20, long-term evolution (LTE), and Ev-DO, HSPA+, HSDPA+, HSUPA+, EDGE, GSM, GPRS, CDMA, TDMA, DECT, and Ethernet derivatives thereof, as well as any other wireless and wired protocols that are designated as 3G, 4G, 5G, and beyond. The computing module may include a plurality of communication modules. For instance, a first communication module may be dedicated to shorter-range wireless communications such as Wi-Fi and Bluetooth, and a second communication module may be dedicated to longer-range wireless communications such as GPS, EDGE, GPRS, CDMA, WiMAX, LTE, Ev-DO, and others.

The modular communication hub 203 may serve as a central connection for one or all of the operating theater devices 1a-1n/2a-2m and handles a data type known as frames. Frames carry the data generated by the devices 1a-1n/2a-2m. When a frame is received by the modular communication hub 203, it is amplified and transmitted to the network router 211, which transfers the data to the cloud computing resources by using a number of wireless or wired communication standards or protocols, as described herein.

The modular communication hub 203 can be used as a standalone device or be connected to compatible network hubs and network switches to form a larger network. The modular communication hub 203 is generally easy to install, configure, and maintain, making it a good option for networking the operating theater devices 1a-1n/2a-2m.

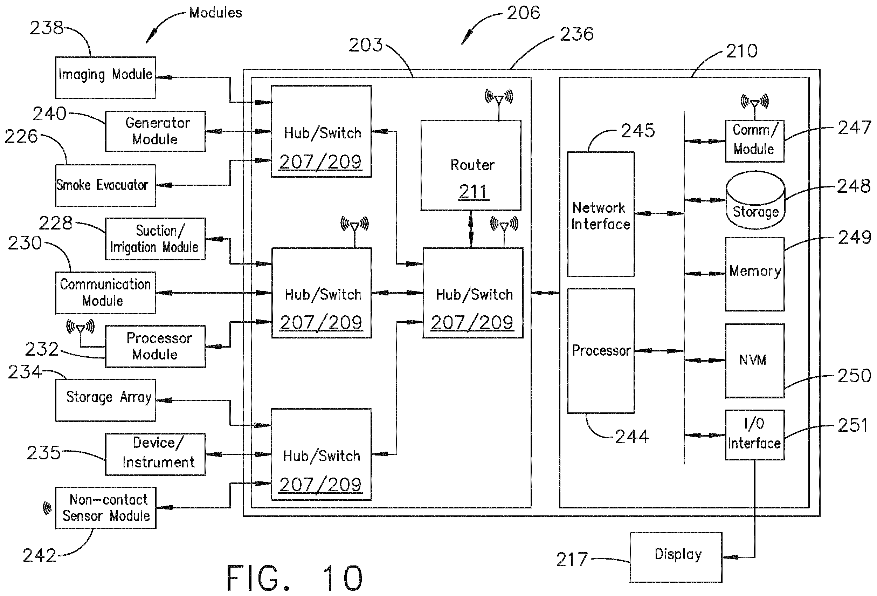

FIG. 9 illustrates a computer-implemented interactive surgical system 200. The computer-implemented interactive surgical system 200 is similar in many respects to the computer-implemented interactive surgical system 100. For example, the computer-implemented interactive surgical system 200 includes one or more surgical systems 202, which are similar in many respects to the surgical systems 102. Each surgical system 202 includes at least one surgical hub 206 in communication with a cloud 204 that may include a remote server 213. In one aspect, the computer-implemented interactive surgical system 200 comprises a modular control tower 236 connected to multiple operating theater devices such as, for example, intelligent surgical instruments, robots, and other computerized devices located in the operating theater. As shown in FIG. 10, the modular control tower 236 comprises a modular communication hub 203 coupled to a computer system 210. As illustrated in the example of FIG. 9, the modular control tower 236 is coupled to an imaging module 238 that is coupled to an endoscope 239, a generator module 240 that is coupled to an energy device 241, a smoke evacuator module 226, a suction/irrigation module 228, a communication module 230, a processor module 232, a storage array 234, a smart device/instrument 235 optionally coupled to a display 237, and a non-contact sensor module 242. The operating theater devices are coupled to cloud computing resources and data storage via the modular control tower 236. A robot hub 222 also may be connected to the modular control tower 236 and to the cloud computing resources. The devices/instruments 235, visualization systems 208, among others, may be coupled to the modular control tower 236 via wired or wireless communication standards or protocols, as described herein. The modular control tower 236 may be coupled to a hub display 215 (e.g., monitor, screen) to display and overlay images received from the imaging module, device/instrument display, and/or other visualization systems 208. The hub display also may display data received from devices connected to the modular control tower in conjunction with images and overlaid images.

FIG. 10 illustrates a surgical hub 206 comprising a plurality of modules coupled to the modular control tower 236. The modular control tower 236 comprises a modular communication hub 203, e.g., a network connectivity device, and a computer system 210 to provide local processing, visualization, and imaging, for example. As shown in FIG. 10, the modular communication hub 203 may be connected in a tiered configuration to expand the number of modules (e.g., devices) that may be connected to the modular communication hub 203 and transfer data associated with the modules to the computer system 210, cloud computing resources, or both. As shown in FIG. 10, each of the network hubs/switches in the modular communication hub 203 includes three downstream ports and one upstream port. The upstream network hub/switch is connected to a processor to provide a communication connection to the cloud computing resources and a local display 217. Communication to the cloud 204 may be made either through a wired or a wireless communication channel.

The surgical hub 206 employs a non-contact sensor module 242 to measure the dimensions of the operating theater and generate a map of the surgical theater using either ultrasonic or laser-type non-contact measurement devices. An ultrasound-based non-contact sensor module scans the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017 in which the sensor module is configured to determine the size of the operating theater and to adjust Bluetooth-pairing distance limits. A laser-based non-contact sensor module scans the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

The computer system 210 comprises a processor 244 and a network interface 245. The processor 244 is coupled to a communication module 247, storage 248, memory 249, non-volatile memory 250, and input/output interface 251 via a system bus. The system bus can be any of several types of bus structure(s) including the memory bus or memory controller, a peripheral bus or external bus, and/or a local bus using any variety of available bus architectures including, but not limited to, 9-bit bus, Industrial Standard Architecture (ISA), Micro-Charmel Architecture (MSA), Extended ISA (EISA), Intelligent Drive Electronics (IDE), VESA Local Bus (VLB), Peripheral Component Interconnect (PCI), USB, Advanced Graphics Port (AGP), Personal Computer Memory Card International Association bus (PCMCIA), Small Computer Systems interface (SCSI), or any other proprietary bus.

The processor 244 may be any single-core or multicore processor such as those known under the trade name ARM Cortex by Texas instruments, In one aspect, the processor may be an LM4F230H5QR ARM Cortex-M4F Processor Core, available from Texas instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare^{®} software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

In one aspect, the processor 244 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The system memory includes volatile memory and non-volatile memory. The basic input/output system (BIOS), containing the basic routines to transfer information between elements within the computer system, such as during start-up, is stored in non-volatile memory. For example, the non-volatile memory can include ROM, programmable ROM (PROM), electrically programmable ROM (EPROM), EEPROM, or flash memory. Volatile memory includes random-access memory (RAM), which acts as external cache memory. Moreover, RAM is available in many forms such as SRAM, dynamic RAM (DRAM), synchronous DRAM (SDRAM), double data rate SDRAM (DDR SDRAM), enhanced SDRAM (ESDRAM), Synchlink DRAM (SLDRAM), and direct Rambus RAM (DRRAM).

The computer system 210 also includes removable/non-removable, volatile/non-volatile computer storage media, such as for example disk storage. The disk storage includes, but is not limited to, devices like a magnetic disk drive, floppy disk drive, tape drive, Jaz drive, Zip drive, LS-60 drive, flash memory card, or memory stick. In addition, the disk storage can include storage media separately or in combination with other storage media including, but not limited to, an optical disc drive such as a compact disc ROM device (CD-ROM), compact disc recordable drive (CD-R Drive), compact disc rewritable drive (CD-RW Drive), or a digital versatile disc ROM drive (DVD-ROM). To facilitate the connection of the disk storage devices to the system bus, a removable or non-removable interface may be employed.

It is to be appreciated that the computer system 210 includes software that acts as an intermediary between users and the basic computer resources described in a suitable operating environment. Such software includes an operating system. The operating system, which can be stored on the disk storage, acts to control and allocate resources of the computer system. System applications take advantage of the management of resources by the operating system through program modules and program data stored either in the system memory or on the disk storage. It is to be appreciated that various components described herein can be implemented with various operating systems or combinations of operating systems.

A user enters commands or information into the computer system 210 through input device(s) coupled to the I/O interface 251. The input devices include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processor through the system bus via interface port(s). The interface port(s) include, for example, a serial port, a parallel port, a game port, and a USB. The output device(s) use some of the same types of ports as input device(s). Thus, for example, a USB port may be used to provide input to the computer system and to output information from the computer system to an output device. An output adapter is provided to illustrate that there are some output devices like monitors, displays, speakers, and printers, among other output devices that require special adapters. The output adapters include, by way of illustration and not limitation, video and sound cards that provide a means of connection between the output device and the system bus. It should be noted that other devices and/or systems of devices, such as remote computer(s), provide both input and output capabilities.

The computer system 210 can operate in a networked environment using logical connections to one or more remote computers, such as cloud computer(s), or local computers. The remote cloud computer(s) can be a personal computer, server, router, network PC, workstation, microprocessor-based appliance, peer device, or other common network node, and the like, and typically includes many or all of the elements described relative to the computer system. For purposes of brevity, only a memory storage device is illustrated with the remote computer(s). The remote computer(s) is logically connected to the computer system through a network interface and then physically connected via a communication connection. The network interface encompasses communication networks such as local area networks (LANs) and wide area networks (WANs). LAN technologies include Fiber Distributed Data Interface (FDDI), Copper Distributed Data Interface (CDDI), Ethernet/IEEE 802.3, Token Ring/IEEE 802.5 and the like. WAN technologies include, but are not limited to, point-to-point links, circuit-switching networks like Integrated Services Digital Networks (ISDN) and variations thereon, packet-switching networks, and Digital Subscriber Lines (DSL).

In various aspects, the computer system 210 of FIG. 10, the imaging module 238 and/or visualization system 208, and/or the processor module 232 of FIGS. 9-10, may comprise an image processor, image-processing engine, media processor, or any specialized digital signal processor (DSP) used for the processing of digital images. The image processor may employ parallel computing with single instruction, multiple data (SIMD) or multiple instruction, multiple data (MIMD) technologies to increase speed and efficiency. The digital image-processing engine can perform a range of tasks. The image processor may be a system on a chip with multicore processor architecture.

The communication connection(s) refers to the hardware/software employed to connect the network interface to the bus. While the communication connection is shown for illustrative clarity inside the computer system, it can also be external to the computer system 210. The hardware/software necessary for connection to the network interface includes, for illustrative purposes only, internal and external technologies such as modems, including regular telephone-grade modems, cable modems, and DSL modems, ISDN adapters, and Ethernet cards.

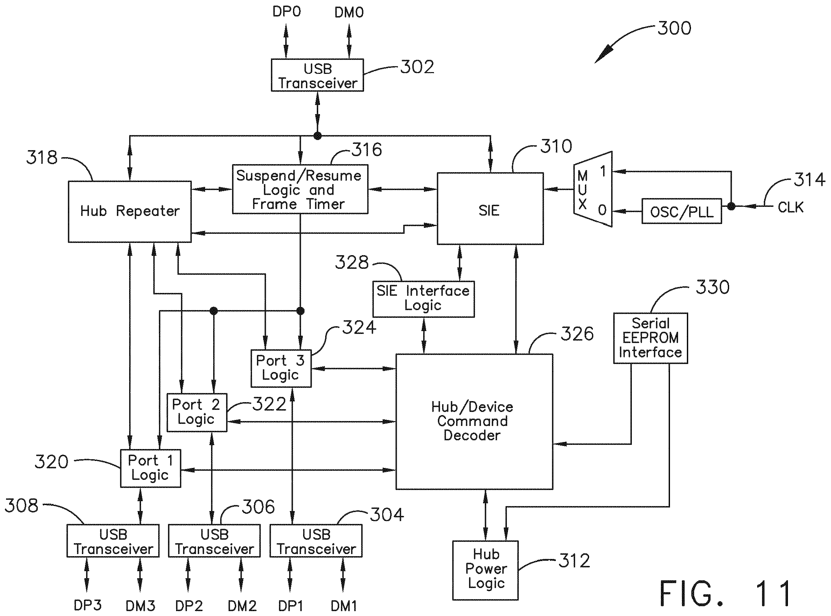

FIG. 11 illustrates a functional block diagram of one aspect of a USB network hub 300 device, in accordance with at least one aspect of the present disclosure. In the illustrated aspect, the USB network hub device 300 employs a TUSB2036 integrated circuit hub by Texas Instruments. The USB network hub 300 is a CMOS device that provides an upstream USB transceiver port 302 and up to three downstream USB transceiver ports 304, 306, 308 in compliance with the USB 2.0 specification. The upstream USB transceiver port 302 is a differential root data port comprising a differential data minus (DM0) input paired with a differential data plus (DP0) input. The three downstream USB transceiver ports 304, 306, 308 are differential data ports where each port includes differential data plus (DP1-DP3) outputs paired with differential data minus (DM1-DM3) outputs.

The USB network hub 300 device is implemented with a digital state machine instead of a microcontroller, and no firmware programming is required. Fully compliant USB transceivers are integrated into the circuit for the upstream USB transceiver port 302 and all downstream USB transceiver ports 304, 306, 308. The downstream USB transceiver ports 304, 306, 308 support both full-speed and low-speed devices by automatically setting the slew rate according to the speed of the device attached to the ports. The USB network hub 300 device may be configured either in bus-powered or self-powered mode and includes a hub power logic 312 to manage power.

The USB network hub 300 device includes a serial interface engine 310 (SIE). The SIE 310 is the front end of the USB network hub 300 hardware and handles most of the protocol described in chapter 8 of the USB specification. The SIE 310 typically comprehends signaling up to the transaction level. The functions that it handles could include: packet recognition, transaction sequencing, SOP, EOP, RESET, and RESUME signal detection/generation, clock/data separation, non-return-to-zero invert (NRZI) data encoding/decoding and bit-stuffing, CRC generation and checking (token and data), packet ID (PID) generation and checking/decoding, and/or serial-parallel/parallel-serial conversion. The 310 receives a clock input 314 and is coupled to a suspend/resume logic and frame timer 316 circuit and a hub repeater circuit 318 to control communication between the upstream USB transceiver port 302 and the downstream USB transceiver ports 304, 306, 308 through port logic circuits 320, 322, 324. The SIE 310 is coupled to a command decoder 326 via interface logic 328 to control commands from a serial EEPROM via a serial EEPROM interface 330.

In various aspects, the USB network hub 300 can connect 127 functions configured in up to six logical layers (tiers) to a single computer. Further, the USB network hub 300 can connect to all peripherals using a standardized four-wire cable that provides both communication and power distribution. The power configurations are bus-powered and self-powered modes. The USB network hub 300 may be configured to support four modes of power management: a bus-powered hub, with either individual-port power management or ganged-port power management, and the self-powered hub, with either individual-port power management or ganged-port power management. In one aspect, using a USB cable, the USB network hub 300, the upstream USB transceiver port 302 is plugged into a USB host controller, and the downstream USB transceiver ports 304, 306, 308 are exposed for connecting USB compatible devices, and so forth.

Additional details regarding the structure and function of the surgical hub and/or surgical hub networks can be found in U.S. Provisional Patent Application No. 62/659,900, titled METHOD OF HUB COMMUNICATION, filed April 19, 2018.

### Cloud System Hardware and Functional Modules

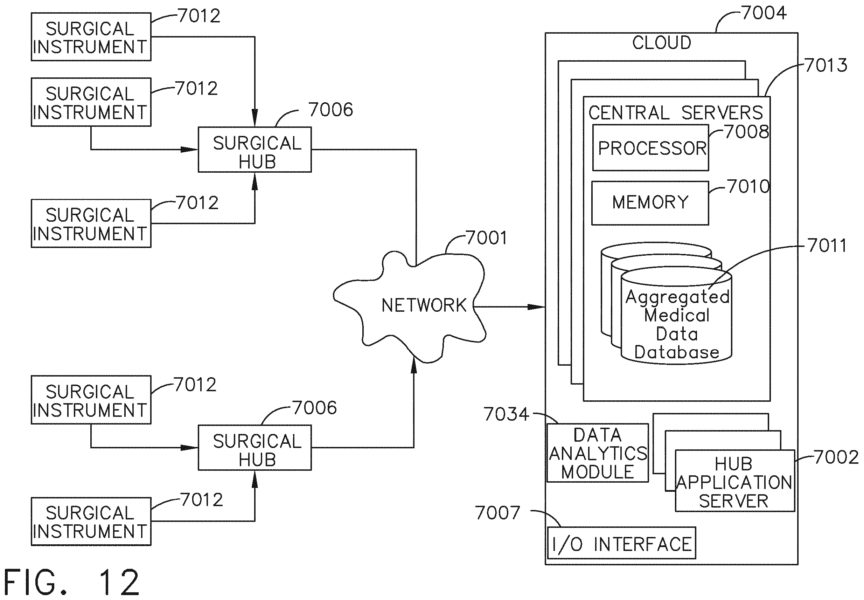

FIG. 12 is a block diagram of the computer-implemented interactive surgical system, in accordance with at least one aspect of the present disclosure. In one aspect, the computer-implemented interactive surgical system is configured to monitor and analyze data related to the operation of various surgical systems that include surgical hubs, surgical instruments, robotic devices and operating theaters or healthcare facilities. The computer-implemented interactive surgical system comprises a cloud-based analytics system. Although the cloud-based analytics system is described as a surgical system, it is not necessarily limited as such and could be a cloud-based medical system generally. As illustrated in FIG. 12, the cloud-based analytics system comprises a plurality of surgical instruments 7012 (may be the same or similar to instruments 112), a plurality of surgical hubs 7006 (may be the same or similar to hubs 106), and a surgical data network 7001 (may be the same or similar to network 201) to couple the surgical hubs 7006 to the cloud 7004 (may be the same or similar to cloud 204). Each of the plurality of surgical hubs 7006 is communicatively coupled to one or more surgical instruments 7012. The hubs 7006 are also communicatively coupled to the cloud 7004 of the computer-implemented interactive surgical system via the network 7001. The cloud 7004 is a remote centralized source of hardware and software for storing, manipulating, and communicating data generated based on the operation of various surgical systems. As shown in FIG. 12, access to the cloud 7004 is achieved via the network 7001, which may be the Internet or some other suitable computer network. Surgical hubs 7006 that are coupled to the cloud 7004 can be considered the client side of the cloud computing system (i.e., cloud-based analytics system). Surgical instruments 7012 are paired with the surgical hubs 7006 for control and implementation of various surgical procedures or operations as described herein.

In addition, surgical instruments 7012 may comprise transceivers for data transmission to and from their corresponding surgical hubs 7006 (which may also comprise transceivers). Combinations of surgical instruments 7012 and corresponding hubs 7006 may indicate particular locations, such as operating theaters in healthcare facilities (e.g., hospitals), for providing medical operations. For example, the memory of a surgical hub 7006 may store location data. As shown in FIG. 12, the cloud 7004 comprises central servers 7013 (which may be same or similar to remote server 113 in FIG. 1 and/or remote server 213 in FIG. 9), hub application servers 7002, data analytics modules 7034, and an input/output ("I/O") interface 7007. The central servers 7013 of the cloud 7004 collectively administer the cloud computing system, which includes monitoring requests by client surgical hubs 7006 and managing the processing capacity of the cloud 7004 for executing the requests. Each of the central servers 7013 comprises one or more processors 7008 coupled to suitable memory devices 7010 which can include volatile memory such as random-access memory (RAM) and non-volatile memory such as magnetic storage devices. The memory devices 7010 may comprise machine executable instructions that when executed cause the processors 7008 to execute the data analytics modules 7034 for the cloud-based data analysis, operations, recommendations and other operations described below. Moreover, the processors 7008 can execute the data analytics modules 7034 independently or in conjunction with hub applications independently executed by the hubs 7006. The central servers 7013 also comprise aggregated medical data databases 2212, which can reside in the memory 2210.

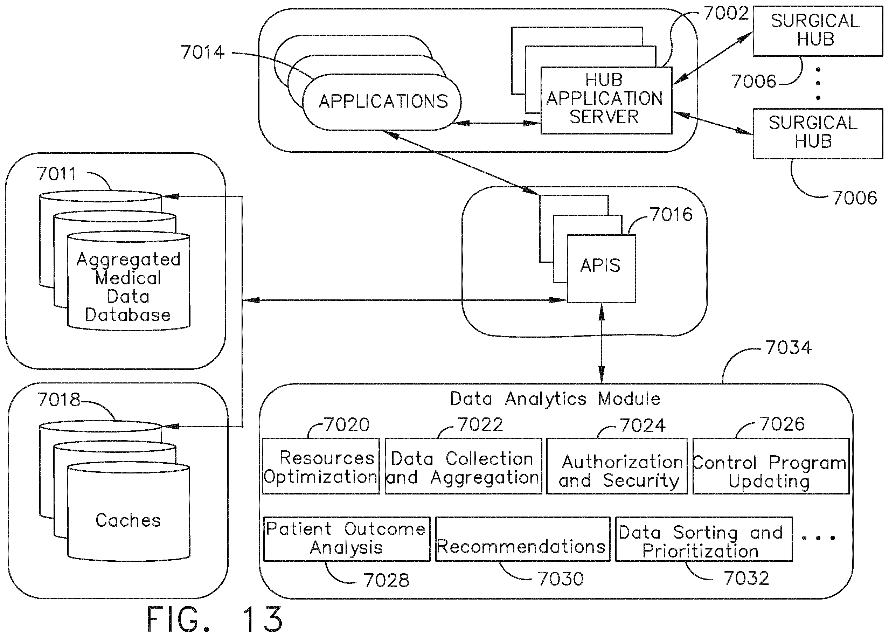

Based on connections to various surgical hubs 7006 via the network 7001, the cloud 7004 can aggregate data from specific data generated by various surgical instruments 7012 and their corresponding hubs 7006. Such aggregated data may be stored within the aggregated medical data databases 7011 of the cloud 7004. In particular, the cloud 7004 may advantageously perform data analysis and operations on the aggregated data to yield insights and/or perform functions that individual hubs 7006 could not achieve on their own. To this end, as shown in FIG. 12, the cloud 7004 and the surgical hubs 7006 are communicatively coupled to transmit and receive information. The I/O interface 7007 is connected to the plurality of surgical hubs 7006 via the network 7001. In this way, the I/O interface 7007 can be configured to transfer information between the surgical hubs 7006 and the aggregated medical data databases 7011. Accordingly, the I/O interface 7007 may facilitate read/write operations of the cloud-based analytics system. Such read/write operations may be executed in response to requests from hubs 7006. These requests could be transmitted to the hubs 7006 through the hub applications. The I/O interface 7007 may include one or more high speed data ports, which may include universal serial bus (USB) ports, IEEE 1394 ports, as well as Wi-Fi and Bluetooth I/O interfaces for connecting the cloud 7004 to hubs 7006. The hub application servers 7002 of the cloud 7004 are configured to host and supply shared capabilities to software applications (e.g. hub applications) executed by surgical hubs 7006. For example, the hub application servers 7002 may manage requests made by the hub applications through the hubs 7006, control access to the aggregated medical data databases 7011, and perform load balancing. The data analytics modules 7034 are described in further detail with reference to FIG. 13.

The particular cloud computing system configuration described in the present disclosure is specifically designed to address various issues arising in the context of medical operations and procedures performed using medical devices, such as the surgical instruments 7012, 112. In particular, the surgical instruments 7012 may be digital surgical devices configured to interact with the cloud 7004 for implementing techniques to improve the performance of surgical operations. Various surgical instruments 7012 and/or surgical hubs 7006 may comprise touch controlled user interfaces such that clinicians may control aspects of interaction between the surgical instruments 7012 and the cloud 7004. Other suitable user interfaces for control such as auditory controlled user interfaces can also be used.

FIG. 13 is a block diagram which illustrates the functional architecture of the computer-implemented interactive surgical system, in accordance with at least one aspect of the present disclosure. The cloud-based analytics system includes a plurality of data analytics modules 7034 that may be executed by the processors 7008 of the cloud 7004 for providing data analytic solutions to problems specifically arising in the medical field. As shown in FIG. 13, the functions of the cloud-based data analytics modules 7034 may be assisted via hub applications 7014 hosted by the hub application servers 7002 that may be accessed on surgical hubs 7006. The cloud processors 7008 and hub applications 7014 may operate in conjunction to execute the data analytics modules 7034. Application program interfaces (APIs) 7016 define the set of protocols and routines corresponding to the hub applications 7014. Additionally, the APIs 7016 manage the storing and retrieval of data into and from the aggregated medical data databases 7011 for the operations of the applications 7014. The caches 7018 also store data (e.g., temporarily) and are coupled to the APIs 7016 for more efficient retrieval of data used by the applications 7014. The data analytics modules 7034 in FIG. 13 include modules for resource optimization 7020, data collection and aggregation 7022, authorization and security 7024, control program updating 7026, patient outcome analysis 7028, recommendations 7030, and data sorting and prioritization 7032. Other suitable data analytics modules could also be implemented by the cloud 7004, according to some aspects. In one aspect, the data analytics modules are used for specific recommendations based on analyzing trends, outcomes, and other data.

For example, the data collection and aggregation module 7022 could be used to generate self-describing data (e.g., metadata) including identification of notable features or configuration (e.g., trends), management of redundant data sets, and storage of the data in paired data sets which can be grouped by surgery but not necessarily keyed to actual surgical dates and surgeons. In particular, pair data sets generated from operations of surgical instruments 7012 can comprise applying a binary classification, e.g., a bleeding or a non-bleeding event. More generally, the binary classification may be characterized as either a desirable event (e.g., a successful surgical procedure) or an undesirable event (e.g., a misfired or misused surgical instrument 7012). The aggregated self-describing data may correspond to individual data received from various groups or subgroups of surgical hubs 7006. Accordingly, the data collection and aggregation module 7022 can generate aggregated metadata or other organized data based on raw data received from the surgical hubs 7006. To this end, the processors 7008 can be operationally coupled to the hub applications 7014 and aggregated medical data databases 7011 for executing the data analytics modules 7034. The data collection and aggregation module 7022 may store the aggregated organized data into the aggregated medical data databases 2212.

The resource optimization module 7020 can be configured to analyze this aggregated data to determine an optimal usage of resources for a particular or group of healthcare facilities. For example, the resource optimization module 7020 may determine an optimal order point of surgical stapling instruments 7012 for a group of healthcare facilities based on corresponding predicted demand of such instruments 7012. The resource optimization module 7020 might also assess the resource usage or other operational configurations of various healthcare facilities to determine whether resource usage could be improved. Similarly, the recommendations module 7030 can be configured to analyze aggregated organized data from the data collection and aggregation module 7022 to provide recommendations. For example, the recommendations module 7030 could recommend to healthcare facilities (e.g., medical service providers such as hospitals) that a particular surgical instrument 7012 should be upgraded to an improved version based on a higher than expected error rate, for example. Additionally, the recommendations module 7030 and/or resource optimization module 7020 could recommend better supply chain parameters such as product reorder points and provide suggestions of different surgical instrument 7012, uses thereof, or procedure steps to improve surgical outcomes. The healthcare facilities can receive such recommendations via corresponding surgical hubs 7006. More specific recommendations regarding parameters or configurations of various surgical instruments 7012 can also be provided. Hubs 7006 and/or surgical instruments 7012 each could also have display screens that display data or recommendations provided by the cloud 7004.

The patient outcome analysis module 7028 can analyze surgical outcomes associated with currently used operational parameters of surgical instruments 7012. The patient outcome analysis module 7028 may also analyze and assess other potential operational parameters. In this connection, the recommendations module 7030 could recommend using these other potential operational parameters based on yielding better surgical outcomes, such as better sealing or less bleeding. For example, the recommendations module 7030 could transmit recommendations to a surgical hub 7006 regarding when to use a particular cartridge for a corresponding stapling surgical instrument 7012. Thus, the cloud-based analytics system, while controlling for common variables, may be configured to analyze the large collection of raw data and to provide centralized recommendations over multiple healthcare facilities (advantageously determined based on aggregated data). For example, the cloud-based analytics system could analyze, evaluate, and/or aggregate data based on type of medical practice, type of patient, number of patients, geographic similarity between medical providers, which medical providers/facilities use similar types of instruments, etc., in a way that no single healthcare facility alone would be able to analyze independently.

The control program updating module 7026 could be configured to implement various surgical instrument 7012 recommendations when corresponding control programs are updated. For example, the patient outcome analysis module 7028 could identify correlations linking specific control parameters with successful (or unsuccessful) results. Such correlations may be addressed when updated control programs are transmitted to surgical instruments 7012 via the control program updating module 7026. Updates to instruments 7012 that are transmitted via a corresponding hub 7006 may incorporate aggregated performance data that was gathered and analyzed by the data collection and aggregation module 7022 of the cloud 7004. Additionally, the patient outcome analysis module 7028 and recommendations module 7030 could identify improved methods of using instruments 7012 based on aggregated performance data.

The cloud-based analytics system may include security features implemented by the cloud 7004. These security features may be managed by the authorization and security module 7024. Each surgical hub 7006 can have associated unique credentials such as username, password, and other suitable security credentials. These credentials could be stored in the memory 7010 and be associated with a permitted cloud access level. For example, based on providing accurate credentials, a surgical hub 7006 may be granted access to communicate with the cloud to a predetermined extent (e.g., may only engage in transmitting or receiving certain defined types of information). To this end, the aggregated medical data databases 7011 of the cloud 7004 may comprise a database of authorized credentials for verifying the accuracy of provided credentials. Different credentials may be associated with varying levels of permission for interaction with the cloud 7004, such as a predetermined access level for receiving the data analytics generated by the cloud 7004.

Furthermore, for security purposes, the cloud could maintain a database of hubs 7006, instruments 7012, and other devices that may comprise a "black list" of prohibited devices. In particular, a surgical hub 7006 listed on the black list may not be permitted to interact with the cloud, while surgical instruments 7012 listed on the black list may not have functional access to a corresponding hub 7006 and/or may be prevented from fully functioning when paired to its corresponding hub 7006. Additionally or alternatively, the cloud 7004 may flag instruments 7012 based on incompatibility or other specified criteria. In this manner, counterfeit medical devices and improper reuse of such devices throughout the cloud-based analytics system can be identified and addressed.

The surgical instruments 7012 may use wireless transceivers to transmit wireless signals that may represent, for example, authorization credentials for access to corresponding hubs 7006 and the cloud 7004. Wired transceivers may also be used to transmit signals. Such authorization credentials can be stored in the respective memory devices of the surgical instruments 7012. The authorization and security module 7024 can determine whether the authorization credentials are accurate or counterfeit. The authorization and security module 7024 may also dynamically generate authorization credentials for enhanced security. The credentials could also be encrypted, such as by using hash based encryption. Upon transmitting proper authorization, the surgical instruments 7012 may transmit a signal to the corresponding hubs 7006 and ultimately the cloud 7004 to indicate that the instruments 7012 are ready to obtain and transmit medical data. In response, the cloud 7004 may transition into a state enabled for receiving medical data for storage into the aggregated medical data databases 7011. This data transmission readiness could be indicated by a light indicator on the instruments 7012, for example. The cloud 7004 can also transmit signals to surgical instruments 7012 for updating their associated control programs. The cloud 7004 can transmit signals that are directed to a particular class of surgical instruments 7012 (e.g., electrosurgical instruments) so that software updates to control programs are only transmitted to the appropriate surgical instruments 7012. Moreover, the cloud 7004 could be used to implement system wide solutions to address local or global problems based on selective data transmission and authorization credentials. For example, if a group of surgical instruments 7012 are identified as having a common manufacturing defect, the cloud 7004 may change the authorization credentials corresponding to this group to implement an operational lockout of the group.

The cloud-based analytics system may allow for monitoring multiple healthcare facilities (e.g., medical facilities like hospitals) to determine improved practices and recommend changes (via the recommendations module 2030, for example) accordingly. Thus, the processors 7008 of the cloud 7004 can analyze data associated with an individual healthcare facility to identify the facility and aggregate the data with other data associated with other healthcare facilities in a group. Groups could be defined based on similar operating practices or geographical location, for example. In this way, the cloud 7004 may provide healthcare facility group wide analysis and recommendations. The cloud-based analytics system could also be used for enhanced situational awareness. For example, the processors 7008 may predictively model the effects of recommendations on the cost and effectiveness for a particular facility (relative to overall operations and/or various medical procedures). The cost and effectiveness associated with that particular facility can also be compared to a corresponding local region of other facilities or any other comparable facilities.

The data sorting and prioritization module 7032 may prioritize and sort data based on criticality (e.g., the severity of a medical event associated with the data, unexpectedness, suspiciousness). This sorting and prioritization may be used in conjunction with the functions of the other data analytics modules 7034 described above to improve the cloud-based analytics and operations described herein. For example, the data sorting and prioritization module 7032 can assign a priority to the data analysis performed by the data collection and aggregation module 7022 and patient outcome analysis modules 7028. Different prioritization levels can result in particular responses from the cloud 7004 (corresponding to a level of urgency) such as escalation for an expedited response, special processing, exclusion from the aggregated medical data databases 7011, or other suitable responses. Moreover, if necessary, the cloud 7004 can transmit a request (e.g. a push message) through the hub application servers for additional data from corresponding surgical instruments 7012. The push message can result in a notification displayed on the corresponding hubs 7006 for requesting supporting or additional data. This push message may be required in situations in which the cloud detects a significant irregularity or outlier and the cloud cannot determine the cause of the irregularity. The central servers 7013 may be programmed to trigger this push message in certain significant circumstances, such as when data is determined to be different from an expected value beyond a predetermined threshold or when it appears security has been comprised, for example.

Additional details regarding the cloud analysis system can be found in U.S. Provisional Patent Application No. 62/659,900, titled METHOD OF HUB COMMUNICATION, filed April 19, 2018.

### Situational Awareness

Although an "intelligent" device including control algorithms that respond to sensed data can be an improvement over a "dumb" device that operates without accounting for sensed data, some sensed data can be incomplete or inconclusive when considered in isolation, i.e., without the context of the type of surgical procedure being performed or the type of tissue that is being operated on. Without knowing the procedural context (e.g., knowing the type of tissue being operated on or the type of procedure being performed), the control algorithm may control the modular device incorrectly or suboptimally given the particular context-free sensed data. For example, the optimal manner for a control algorithm to control a surgical instrument in response to a particular sensed parameter can vary according to the particular tissue type being operated on. This is due to the fact that different tissue types have different properties (e.g., resistance to tearing) and thus respond differently to actions taken by surgical instruments. Therefore, it may be desirable for a surgical instrument to take different actions even when the same measurement for a particular parameter is sensed. As one specific example, the optimal manner in which to control a surgical stapling and cutting instrument in response to the instrument sensing an unexpectedly high force to close its end effector will vary depending upon whether the tissue type is susceptible or resistant to tearing. For tissues that are susceptible to tearing, such as lung tissue, the instrument's control algorithm would optimally ramp down the motor in response to an unexpectedly high force to close to avoid tearing the tissue. For tissues that are resistant to tearing, such as stomach tissue, the instrument's control algorithm would optimally ramp up the motor in response to an unexpectedly high force to close to ensure that the end effector is clamped properly on the tissue. Without knowing whether lung or stomach tissue has been clamped, the control algorithm may make a suboptimal decision.

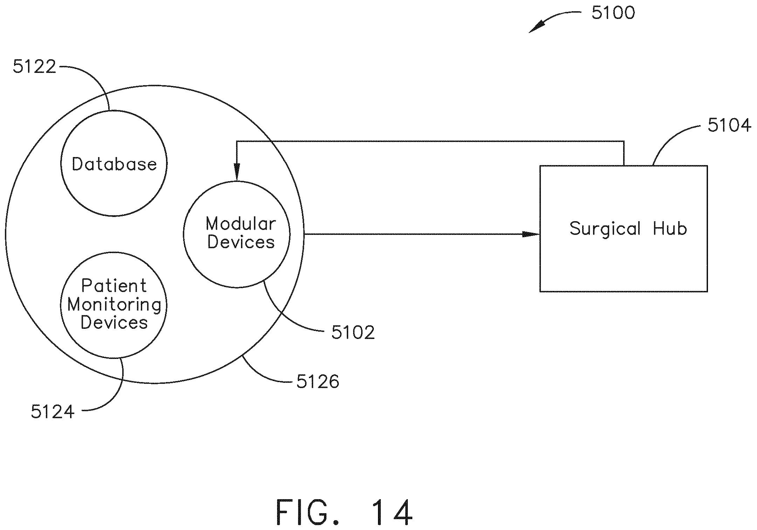

One solution utilizes a surgical hub including a system that is configured to derive information about the surgical procedure being performed based on data received from various data sources and then control the paired modular devices accordingly. In other words, the surgical hub is configured to infer information about the surgical procedure from received data and then control the modular devices paired to the surgical hub based upon the inferred context of the surgical procedure. FIG. 14 illustrates a diagram of a situationally aware surgical system 5100, in accordance with at least one aspect of the present disclosure. In some exemplifications, the data sources 5126 include, for example, the modular devices 5102 (which can include sensors configured to detect parameters associated with the patient and/or the modular device itself), databases 5122 (e.g., an EMR database containing patient records), and patient monitoring devices 5124 (e.g., a blood pressure (BP) monitor and an electrocardiography (EKG) monitor).

A surgical hub 5104, which may be similar to the hub 106 in many respects, can be configured to derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." In one exemplification, the surgical hub 5104 can incorporate a situational awareness system, which is the hardware and/or programming associated with the surgical hub 5104 that derives contextual information pertaining to the surgical procedure from the received data.

The situational awareness system of the surgical hub 5104 can be configured to derive the contextual information from the data received from the data sources 5126 in a variety of different ways. In one exemplification, the situational awareness system includes a pattern recognition system, or machine learning system (e.g., an artificial neural network), that has been trained on training data to correlate various inputs (e.g., data from databases 5122, patient monitoring devices 5124, and/or modular devices 5102) to corresponding contextual information regarding a surgical procedure. In other words, a machine learning system can be trained to accurately derive contextual information regarding a surgical procedure from the provided inputs. In another exemplification, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In one exemplification, the contextual information received by the situational awareness system of the surgical hub 5104 is associated with a particular control adjustment or set of control adjustments for one or more modular devices 5102. In another exemplification, the situational awareness system includes a further machine learning system, lookup table, or other such system, which generates or retrieves one or more control adjustments for one or more modular devices 5102 when provided the contextual information as input.

A surgical hub 5104 incorporating a situational awareness system provides a number of benefits for the surgical system 5100. One benefit includes improving the interpretation of sensed and collected data, which would in turn improve the processing accuracy and/or the usage of the data during the course of a surgical procedure. To return to a previous example, a situationally aware surgical hub 5104 could determine what type of tissue was being operated on; therefore, when an unexpectedly high force to close the surgical instrument's end effector is detected, the situationally aware surgical hub 5104 could correctly ramp up or ramp down the motor of the surgical instrument for the type of tissue.

As another example, the type of tissue being operated can affect the adjustments that are made to the compression rate and load thresholds of a surgical stapling and cutting instrument for a particular tissue gap measurement. A situationally aware surgical hub 5104 could infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The surgical hub 5104 could then adjust the compression rate and load thresholds of the surgical stapling and cutting instrument appropriately for the type of tissue.

As yet another example, the type of body cavity being operated in during an insufflation procedure can affect the function of a smoke evacuator. A situationally aware surgical hub 5104 could determine whether the surgical site is under pressure (by determining that the surgical procedure is utilizing insufflation) and determine the procedure type. As a procedure type is generally performed in a specific body cavity, the surgical hub 5104 could then control the motor rate of the smoke evacuator appropriately for the body cavity being operated in. Thus, a situationally aware surgical hub 5104 could provide a consistent amount of smoke evacuation for both thoracic and abdominal procedures.

As yet another example, the type of procedure being performed can affect the optimal energy level for an ultrasonic surgical instrument or radio frequency (RF) electrosurgical instrument to operate at. Arthroscopic procedures, for example, require higher energy levels because the end effector of the ultrasonic surgical instrument or RF electrosurgical instrument is immersed in fluid. A situationally aware surgical hub 5104 could determine whether the surgical procedure is an arthroscopic procedure. The surgical hub 5104 could then adjust the RF power level or the ultrasonic amplitude of the generator (i.e., "energy level") to compensate for the fluid filled environment. Relatedly, the type of tissue being operated on can affect the optimal energy level for an ultrasonic surgical instrument or RF electrosurgical instrument to operate at. A situationally aware surgical hub 5104 could determine what type of surgical procedure is being performed and then customize the energy level for the ultrasonic surgical instrument or RF electrosurgical instrument, respectively, according to the expected tissue profile for the surgical procedure. Furthermore, a situationally aware surgical hub 5104 can be configured to adjust the energy level for the ultrasonic surgical instrument or RF electrosurgical instrument throughout the course of a surgical procedure, rather than just on a procedure-by-procedure basis. A situationally aware surgical hub 5104 could determine what step of the surgical procedure is being performed or will subsequently be performed and then update the control algorithms for the generator and/or ultrasonic surgical instrument or RF electrosurgical instrument to set the energy level at a value appropriate for the expected tissue type according to the surgical procedure step.

As yet another example, data can be drawn from additional data sources 5126 to improve the conclusions that the surgical hub 5104 draws from one data source 5126. A situationally aware surgical hub 5104 could augment data that it receives from the modular devices 5102 with contextual information that it has built up regarding the surgical procedure from other data sources 5126. For example, a situationally aware surgical hub 5104 can be configured to determine whether hemostasis has occurred (i.e., whether bleeding at a surgical site has stopped) according to video or image data received from a medical imaging device. However, in some cases the video or image data can be inconclusive. Therefore, in one exemplification, the surgical hub 5104 can be further configured to compare a physiologic measurement (e.g., blood pressure sensed by a BP monitor communicably connected to the surgical hub 5104) with the visual or image data of hemostasis (e.g., from a medical imaging device 124 (FIG. 2) communicably coupled to the surgical hub 5104) to make a determination on the integrity of the staple line or tissue weld. In other words, the situational awareness system of the surgical hub 5104 can consider the physiological measurement data to provide additional context in analyzing the visualization data. The additional context can be useful when the visualization data may be inconclusive or incomplete on its own.

Another benefit includes proactively and automatically controlling the paired modular devices 5102 according to the particular step of the surgical procedure that is being performed to reduce the number of times that medical personnel are required to interact with or control the surgical system 5100 during the course of a surgical procedure. For example, a situationally aware surgical hub 5104 could proactively activate the generator to which an RF electrosurgical instrument is connected if it determines that a subsequent step of the procedure requires the use of the instrument. Proactively activating the energy source allows the instrument to be ready for use a soon as the preceding step of the procedure is completed.

As another example, a situationally aware surgical hub 5104 could determine whether the current or subsequent step of the surgical procedure requires a different view or degree of magnification on the display according to the feature(s) at the surgical site that the surgeon is expected to need to view. The surgical hub 5104 could then proactively change the displayed view (supplied by, e.g., a medical imaging device for the visualization system 108) accordingly so that the display automatically adjusts throughout the surgical procedure.

As yet another example, a situationally aware surgical hub 5104 could determine which step of the surgical procedure is being performed or will subsequently be performed and whether particular data or comparisons between data will be required for that step of the surgical procedure. The surgical hub 5104 can be configured to automatically call up data screens based upon the step of the surgical procedure being performed, without waiting for the surgeon to ask for the particular information.

Another benefit includes checking for errors during the setup of the surgical procedure or during the course of the surgical procedure. For example, a situationally aware surgical hub 5104 could determine whether the operating theater is setup properly or optimally for the surgical procedure to be performed. The surgical hub 5104 can be configured to determine the type of surgical procedure being performed, retrieve the corresponding checklists, product location, or setup needs (e.g., from a memory), and then compare the current operating theater layout to the standard layout for the type of surgical procedure that the surgical hub 5104 determines is being performed. In one exemplification, the surgical hub 5104 can be configured to compare the list of items for the procedure scanned by a suitable scanner for example and/or a list of devices paired with the surgical hub 5104 to a recommended or anticipated manifest of items and/or devices for the given surgical procedure. If there are any discontinuities between the lists, the surgical hub 5104 can be configured to provide an alert indicating that a particular modular device 5102, patient monitoring device 5124, and/or other surgical item is missing. In one exemplification, the surgical hub 5104 can be configured to determine the relative distance or position of the modular devices 5102 and patient monitoring devices 5124 via proximity sensors, for example. The surgical hub 5104 can compare the relative positions of the devices to a recommended or anticipated layout for the particular surgical procedure. If there are any discontinuities between the layouts, the surgical hub 5104 can be configured to provide an alert indicating that the current layout for the surgical procedure deviates from the recommended layout.

As another example, a situationally aware surgical hub 5104 could determine whether the surgeon (or other medical personnel) was making an error or otherwise deviating from the expected course of action during the course of a surgical procedure. For example, the surgical hub 5104 can be configured to determine the type of surgical procedure being performed, retrieve the corresponding list of steps or order of equipment usage (e.g., from a memory), and then compare the steps being performed or the equipment being used during the course of the surgical procedure to the expected steps or equipment for the type of surgical procedure that the surgical hub 5104 determined is being performed. In one exemplification, the surgical hub 5104 can be configured to provide an alert indicating that an unexpected action is being performed or an unexpected device is being utilized at the particular step in the surgical procedure.

Overall, the situational awareness system for the surgical hub 5104 improves surgical procedure outcomes by adjusting the surgical instruments (and other modular devices 5102) for the particular context of each surgical procedure (such as adjusting to different tissue types) and validating actions during a surgical procedure. The situational awareness system also improves surgeons' efficiency in performing surgical procedures by automatically suggesting next steps, providing data, and adjusting displays and other modular devices 5102 in the surgical theater according to the specific context of the procedure.

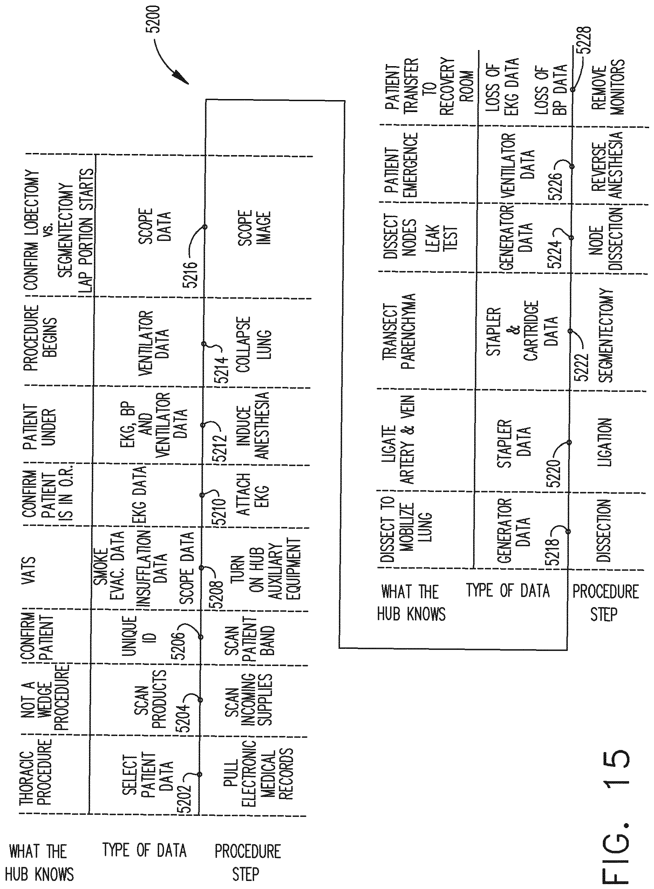

Referring now to FIG. 15, a timeline 5200 depicting situational awareness of a hub, such as the surgical hub 106 or 206 (FIGS. 1-11), for example, is depicted. The timeline 5200 is an illustrative surgical procedure and the contextual information that the surgical hub 106, 206 can derive from the data received from the data sources at each step in the surgical procedure. The timeline 5200 depicts the typical steps that would be taken by the nurses, surgeons, and other medical personnel during the course of a lung segmentectomy procedure, beginning with setting up the operating theater and ending with transferring the patient to a post-operative recovery room.

The situationally aware surgical hub 106, 206 receives data from the data sources throughout the course of the surgical procedure, including data generated each time medical personnel utilize a modular device that is paired with the surgical hub 106, 206. The surgical hub 106, 206 can receive this data from the paired modular devices and other data sources and continually derive inferences (i.e., contextual information) about the ongoing procedure as new data is received, such as which step of the procedure is being performed at any given time. The situational awareness system of the surgical hub 106, 206 is able to, for example, record data pertaining to the procedure for generating reports, verify the steps being taken by the medical personnel, provide data or prompts (e.g., via a display screen) that may be pertinent for the particular procedural step, adjust modular devices based on the context (e.g., activate monitors, adjust the field of view (FOV) of the medical imaging device, or change the energy level of an ultrasonic surgical instrument or RF electrosurgical instrument), and take any other such action described above.

As the first step 5202 in this illustrative procedure, the hospital staff members retrieve the patient's EMR from the hospital's EMR database. Based on select patient data in the EMR, the surgical hub 106, 206 determines that the procedure to be performed is a thoracic procedure.

Second step 5204, the staff members scan the incoming medical supplies for the procedure. The surgical hub 106, 206 cross-references the scanned supplies with a list of supplies that are utilized in various types of procedures and confirms that the mix of supplies corresponds to a thoracic procedure. Further, the surgical hub 106, 206 is also able to determine that the procedure is not a wedge procedure (because the incoming supplies either lack certain supplies that are necessary for a thoracic wedge procedure or do not otherwise correspond to a thoracic wedge procedure).

Third step 5206, the medical personnel scan the patient band via a scanner that is communicably connected to the surgical hub 106, 206. The surgical hub 106, 206 can then confirm the patient's identity based on the scanned data.

Fourth step 5208, the medical staff turns on the auxiliary equipment. The auxiliary equipment being utilized can vary according to the type of surgical procedure and the techniques to be used by the surgeon, but in this illustrative case they include a smoke evacuator, insufflator, and medical imaging device. When activated, the auxiliary equipment that are modular devices can automatically pair with the surgical hub 106, 206 that is located within a particular vicinity of the modular devices as part of their initialization process. The surgical hub 106, 206 can then derive contextual information about the surgical procedure by detecting the types of modular devices that pair with it during this pre-operative or initialization phase. In this particular example, the surgical hub 106, 206 determines that the surgical procedure is a VATS procedure based on this particular combination of paired modular devices. Based on the combination of the data from the patient's EMR, the list of medical supplies to be used in the procedure, and the type of modular devices that connect to the hub, the surgical hub 106, 206 can generally infer the specific procedure that the surgical team will be performing. Once the surgical hub 106, 206 knows what specific procedure is being performed, the surgical hub 106, 206 can then retrieve the steps of that procedure from a memory or from the cloud and then cross-reference the data it subsequently receives from the connected data sources (e.g., modular devices and patient monitoring devices) to infer what step of the surgical procedure the surgical team is performing.

Fifth step 5210, the staff members attach the EKG electrodes and other patient monitoring devices to the patient. The EKG electrodes and other patient monitoring devices are able to pair with the surgical hub 106, 206. As the surgical hub 106, 206 begins receiving data from the patient monitoring devices, the surgical hub 106, 206 thus confirms that the patient is in the operating theater.

Sixth step 5212, the medical personnel induce anesthesia in the patient. The surgical hub 106, 206 can infer that the patient is under anesthesia based on data from the modular devices and/or patient monitoring devices, including EKG data, blood pressure data, ventilator data, or combinations thereof, for example. Upon completion of the sixth step 5212, the pre-operative portion of the lung segmentectomy procedure is completed and the operative portion begins.

Seventh step 5214, the patient's lung that is being operated on is collapsed (while ventilation is switched to the contralateral lung). The surgical hub 106, 206 can infer from the ventilator data that the patient's lung has been collapsed, for example. The surgical hub 106, 206 can infer that the operative portion of the procedure has commenced as it can compare the detection of the patient's lung collapsing to the expected steps of the procedure (which can be accessed or retrieved previously) and thereby determine that collapsing the lung is the first operative step in this particular procedure.

Eighth step 5216, the medical imaging device (e.g., a scope) is inserted and video from the medical imaging device is initiated. The surgical hub 106, 206 receives the medical imaging device data (i.e., video or image data) through its connection to the medical imaging device. Upon receipt of the medical imaging device data, the surgical hub 106, 206 can determine that the laparoscopic portion of the surgical procedure has commenced. Further, the surgical hub 106, 206 can determine that the particular procedure being performed is a segmentectomy, as opposed to a lobectomy (note that a wedge procedure has already been discounted by the surgical hub 106, 206 based on data received at the second step 5204 of the procedure). The data from the medical imaging device 124 (FIG. 2) can be utilized to determine contextual information regarding the type of procedure being performed in a number of different ways, including by determining the angle at which the medical imaging device is oriented with respect to the visualization of the patient's anatomy, monitoring the number or medical imaging devices being utilized (i.e., that are activated and paired with the surgical hub 106, 206), and monitoring the types of visualization devices utilized. For example, one technique for performing a VATS lobectomy places the camera in the lower anterior corner of the patient's chest cavity above the diaphragm, whereas one technique for performing a VATS segmentectomy places the camera in an anterior intercostal position relative to the segmental fissure. Using pattern recognition or machine learning techniques, for example, the situational awareness system can be trained to recognize the positioning of the medical imaging device according to the visualization of the patient's anatomy. As another example, one technique for performing a VATS lobectomy utilizes a single medical imaging device, whereas another technique for performing a VATS segmentectomy utilizes multiple cameras. As yet another example, one technique for performing a VATS segmentectomy utilizes an infrared light source (which can be communicably coupled to the surgical hub as part of the visualization system) to visualize the segmental fissure, which is not utilized in a VATS lobectomy. By tracking any or all of this data from the medical imaging device, the surgical hub 106, 206 can thereby determine the specific type of surgical procedure being performed and/or the technique being used for a particular type of surgical procedure.

Ninth step 5218, the surgical team begins the dissection step of the procedure. The surgical hub 106, 206 can infer that the surgeon is in the process of dissecting to mobilize the patient's lung because it receives data from the RF or ultrasonic generator indicating that an energy instrument is being fired. The surgical hub 106, 206 can cross-reference the received data with the retrieved steps of the surgical procedure to determine that an energy instrument being fired at this point in the process (i.e., after the completion of the previously discussed steps of the procedure) corresponds to the dissection step. In certain instances, the energy instrument can be an energy tool mounted to a robotic arm of a robotic surgical system.

Tenth step 5220, the surgical team proceeds to the ligation step of the procedure. The surgical hub 106, 206 can infer that the surgeon is ligating arteries and veins because it receives data from the surgical stapling and cutting instrument indicating that the instrument is being fired. Similarly to the prior step, the surgical hub 106, 206 can derive this inference by cross-referencing the receipt of data from the surgical stapling and cutting instrument with the retrieved steps in the process. In certain instances, the surgical instrument can be a surgical tool mounted to a robotic arm of a robotic surgical system.

Eleventh step 5222, the segmentectomy portion of the procedure is performed. The surgical hub 106, 206 can infer that the surgeon is transecting the parenchyma based on data from the surgical stapling and cutting instrument, including data from its cartridge. The cartridge data can correspond to the size or type of staple being fired by the instrument, for example. As different types of staples are utilized for different types of tissues, the cartridge data can thus indicate the type of tissue being stapled and/or transected. In this case, the type of staple being fired is utilized for parenchyma (or other similar tissue types), which allows the surgical hub 106, 206 to infer that the segmentectomy portion of the procedure is being performed.

Twelfth step 5224, the node dissection step is then performed. The surgical hub 106, 206 can infer that the surgical team is dissecting the node and performing a leak test based on data received from the generator indicating that an RF or ultrasonic instrument is being fired. For this particular procedure, an RF or ultrasonic instrument being utilized after parenchyma was transected corresponds to the node dissection step, which allows the surgical hub 106, 206 to make this inference. It should be noted that surgeons regularly switch back and forth between surgical stapling/cutting instruments and surgical energy (i.e., RF or ultrasonic) instruments depending upon the particular step in the procedure because different instruments are better adapted for particular tasks. Therefore, the particular sequence in which the stapling/cutting instruments and surgical energy instruments are used can indicate what step of the procedure the surgeon is performing. Moreover, in certain instances, robotic tools can be utilized for one or more steps in a surgical procedure and/or handheld surgical instruments can be utilized for one or more steps in the surgical procedure. The surgeon(s) can alternate between robotic tools and handheld surgical instruments and/or can use the devices concurrently, for example. Upon completion of the twelfth step 5224, the incisions are closed up and the post-operative portion of the procedure begins.

Thirteenth step 5226, the patient's anesthesia is reversed. The surgical hub 106, 206 can infer that the patient is emerging from the anesthesia based on the ventilator data (i.e., the patient's breathing rate begins increasing), for example.

Lastly, the fourteenth step 5228 is that the medical personnel remove the various patient monitoring devices from the patient. The surgical hub 106, 206 can thus infer that the patient is being transferred to a recovery room when the hub loses EKG, BP, and other data from the patient monitoring devices. As can be seen from the description of this illustrative procedure, the surgical hub 106, 206 can determine or infer when each step of a given surgical procedure is taking place according to data received from the various data sources that are communicably coupled to the surgical hub 106, 206.

Situational awareness is further described in U.S. Provisional Patent Application Serial No. 62/659,900, titled METHOD OF HUB COMMUNICATION, filed April 19, 2018. In certain instances, operation of a robotic surgical system, including the various robotic surgical systems disclosed herein, for example, can be controlled by the hub 106, 206 based on its situational awareness and/or feedback from the components thereof and/or based on information from the cloud 104.

### Structured Data Sharing

A variety of computer systems have been described herein, including surgical hubs 106, 206 (FIGS. 1-11) and various computing systems to which the surgical hubs 106, 206 are communicably connectable, including cloud computing systems 104, 204, 7004 (FIGS. 1, 9, and 12-13). In other implementations, surgical hubs 106, 206 can be communicably connected to each other or to various databases within or associated with a medical facility to form local computer system networks. In each of these various aspects, the surgical hubs 106, 206, databases, and other computer systems generate and utilize substantial amounts of data related to patients, surgical procedures, surgical staff, and so on. Therefore, it can be beneficial for the surgical hubs 106, 206 and other computer systems to share data with each other in an efficient and structured manner. Accordingly, computer systems described herein can be configured to aggregate and share data collected both within the operating room (OR) and throughout the medical facility in order to perform analyses of OR operations and efficiency, patient outcomes, surgical staff performance, and so on. In sum, the systems and techniques described herein can be utilized for facility-wide collection and interpretation of data.

A variety of paradigms or techniques can be utilized to efficiently share data between interrelated or connected databases, such as implementing relational database models or utilizing consistent data formats so that data is portable across the different computer systems in a network. Two general structured data-sharing paradigms described herein are referred to as "data interoperability" and "data fluidity." These data-sharing paradigms can be characterized as rulesets executed by each of the computer systems within a computer network that define how and in what ways data is shared by and between the computer systems within the computer network. The rule set can be embodied as a set of computer-executable instructions stored in a memory of a computer system (e.g., memory 249 of the surgical hub 206 illustrated in FIG. 10) that, when executed by a processor (e.g., processor 244), cause the computer system to perform the described steps for sharing data with other connected computer systems. Further, all of the databases described herein can be stored in a memory (e.g., memory 249) of a computer system, such as a surgical hub 106, 206 or a database server. When it is stated herein that databases communicate or share data with each other, what is meant is that the computer system storing the databases are creating, updating, retrieving, and/or administering the data within the databases as described. Further, access to and control of the databases can be managed by a database management system executed by the computer systems, which can include computer-executable instructions stored in the memory (e.g., memory 249) of the computer system that allow users to interact with the various databases and for data to be communicated by and between the various databases.

Data interoperability is defined as the ability of computer or database systems to work cooperatively by having a database automatically transmit particular data to recipient databases according to predefined rules. For each type of data generated by or at a computer system, the rules of the data interoperability paradigm delineate to which recipient database(s) the computer should transmit each type of data and, in some cases, the data format each type of data is to be transmitted in to each particular recipient database. In some aspects, data interoperability can be characterized as a one-way communication of data between computer systems. Further, in some aspects, the computer system transmitting data through the one-way communication channel can lack the ability to accept data of the same type back from the receiving computer system. These aspects can be beneficial in order to, for example, have one database drive or control the data that is stored or presented in another database.

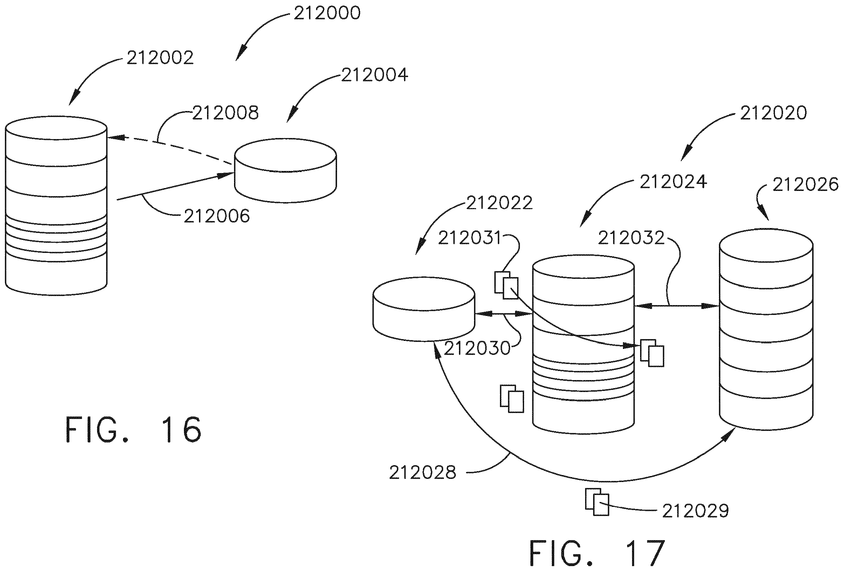

Illustrative of these concepts, FIG. 16 is a diagram of a database system 212000 illustrating data interoperability between interrelated databases, in accordance with at least one aspect of the present disclosure. In the depicted aspect, the database system 212000 includes a first database 212002 communicably connected to a second database 212004. In one aspect, the first database 212002 can be programmed to transmit data to the second database 212004 in a solely or primarily unidirectional manner. In other words, data updates or new data flow from the first database 212002 to the second database 212004, but not vice versa. In some aspects, all of the data stored in the first database 212002 can be restricted to a unidirectional data flow between the databases 212002, 212004. In other aspects, a particular type or subset of data stored in the first database 212002 can be restricted to a unidirectional data flow between the databases 212002, 212004.

For example, the first database 212002 can include an EHR database, and the second database 212004 can include a pharmacy database. In this implementation, the data interoperability ruleset can dictate that when a patient's EHR is updated in the EHR database to indicate that a new medication has been prescribed to the patient, the relevant prescription data can be automatically transmitted to the pharmacy database as a new prescription request for processing by the pharmacy department. Accordingly, the first database 212002 can be programmed to transmit 212006 data representing a prescription request to the second database 212004. The data in the prescription request can include, for example, drug interaction data and a current drug list from the associated patient's EHRs. Further, the data interoperability ruleset can dictate that when a prescription is prepared in response to a received prescription request, a billing update can be automatically transmitted to the EHR database. Accordingly, the second database 212004 can be programmed to transmit 212008 data representing a billing update to the first database 212002 in response to or upon fulfillment of the prescription request. The transmission of each of these types of data can be unidirectional with respect to the respective databases 212002, 212004.

As another example, the first database 212002 can include an OR scheduling database, and the second database 212004 can include a medical supply database. In this implementation, the data interoperability ruleset can dictate that when a new operation is scheduled or input into the OR scheduling database, relevant data for the scheduled operation can be automatically transmitted to the medical supply database to indicate which supplies should be prepared by the medical supply department and at what time and date they should be prepared by. Accordingly, the OR scheduling database can automatically transmit 212006 data representing a procedure to the medical supply database when a new procedure is scheduled. Accordingly, the employees with access to the medical supply database can automatically receive updates so that they can have the products and instruments needed for the scheduled procedure prepared at the scheduled time.

As yet another example, the first database 212002 can include a lab database, and the second database 212004 can include an EHR database. In this implementation, the data interoperability ruleset can dictate that when a patient's lab results are uploaded to the lab database, the lab results data can be automatically transmitted to the EHR database to be associated with the patient's EHR. Accordingly, the lab database can automatically populate the EHR database with data representing test results and labs when they are completed. Accordingly, physicians and any other individuals with access to the patient EHR can immediately access the results of any ordered tests and labs without the need to take any further action.

As yet another example, the first database 212002 can include a prescription-entering or EHR database, and the second database 212004 can include a medication-dispensing or pharmacy database. In this implementation, the data interoperability ruleset can dictate that when a new prescription is entered for a patient, the relevant prescription data can be automatically transmitted to the pharmacy database as a new prescription request for processing by the pharmacy department. Accordingly, the medication-dispensing database can automatically receive the prescription when entered by the practitioner so that the prescription can be ready as needed.

As yet another example, the first database 212002 can include a pathology database, and the second database 212004 can include an OR database (e.g., stored in a surgical hub 106, 206). In this implementation, the data interoperability ruleset can dictate that when new pathology results are received for a patient, the relevant pathology data can automatically be transmitted to the OR database for review by the surgical staff. Accordingly, data including updates or results stored in the pathology database can be automatically transmitted 212006 to the OR through an update to the OR database. The data can be transmitted 212006 between the pathology database and the OR database in real time, such as during the course or a surgical procedure to inform subsequent steps of the procedure. As a specific illustration, during a wedge resection procedure to remove a small tumor in a patient's lung, the surgical staff sends the resected specimen to the pathology department to check for malignancy while the patient is still in the OR. If the pathology department confirms malignancy, the surgical staff often elects to complete a lobectomy procedure on the lobe from which the wedge was taken. Accordingly, this process of providing notifications from other departments to the surgical staff during the course of a surgical procedure via the surgical hub can be automated by utilizing a data interoperability paradigm between the pathology database and the surgical hubs, as described above.

Data fluidity is defined as the ability of data to flow from one database to another database according to predefined rules that delineate bidirectional relationships between databases for data sets stored therein. In some aspects, the data fluidity paradigm can define whether data is transmitted to particular recipient databases and/or whether data is linked to particular recipient databases. Data can be automatically shared with or transferred to other databases utilizing relational database techniques (i.e., relations defined between the databases), for example. In one aspect, the databases can execute a set of rules that define which types of data are to be automatically transmitted to which particular recipient database. Furthermore, in one aspect, the databases can execute a set of rules that define the format of the data or the database to which the data is transmitted according to surgical contextual data (metadata) associated with the data. The ruleset can be embodied as a set of computer-executable instructions stored in a memory of a computer system (e.g., memory 249 of the surgical hub 206 illustrated in FIG. 10) that, when executed by a processor (e.g., processor 244), cause the computer system to perform the described steps for sharing data with other connected computer systems.

For example, a surgical hub can utilize situational awareness (described above under the heading SITUATIONAL AWARENESS) to determine the surgical context (e.g., the surgical procedure type or the surgical procedure step being performed) based on the perioperative data received from the surgical instrument, patient monitors, and other surgical devices or databases and then associate the surgical context with the data being generated (e.g., store the surgical context as metadata for the generated data). The determined surgical context can influence which particular database(s) receive particular data, how much of the data is transmitted to the recipient database(s), the data format in which the data is transmitted, and so on. Accordingly, the computer system (e.g., a surgical hub) can then transmit the gathered data (with or without its associated surgical metadata) to particular recipient databases or in particular data formats according to the determined surgical context. In various aspects, the surgical context can influence the bit size, quantity, resolution, and/or time bracket around the transmitted data (e.g., the number of samples of the data captured at a particular sampling rate). Accordingly, the data fluidity paradigm allows interrelated databases to share data relevant to each database according to the needs of each recipient database. In other words, computer systems sharing data according to a data fluidity paradigm can anticipate the potential uses and needs for data received by the computer systems and then automatically route data to recipient databases or computer systems accordingly. Further, the surgical context can dictate the format that a computer system transmits the data in, the breadth of the data transmitted by the computer system, and so on.

Illustrative of these concepts, FIG. 17 is a diagram of a database system illustrating data fluidity between interrelated databases, in accordance with at least one aspect of the present disclosure. In the depicted aspect, the database system 212020 includes a first database 212022, a second database 212024, and a third database 212026 that are each communicably connected together. In one aspect, each of the databases 212022, 212024, 212026 is programmed to communicate data in a bidirectional manner. In other words, when a particular data set in one of the databases 212022, 212024, 212026 is updated and the updated data is relevant to another of the databases 212022, 212024, 212026 (as dictated by the particular data fluidity rules defining the relationships between the databases 212022, 212024, 212026), the database 212022, 212024, 212026 at which the data was updated can automatically share or transmit those updates to the corresponding database(s) 212022, 212024, 212026.

The data fluidity rulesets dictating data flow between different databases can be defined (e.g., by administrators of the database system 212020) according to the relationships between the departments represented by the databases 212022, 212024, 212026. For example, some departments (e.g., OR and pathology or OR and supply) routinely collaborate or consult with each other on medical issues occurring with patients in the medical facility. Accordingly, the data fluidity rules can dictate that when an update is made to a particular data type (or a set of data types) in one of these collaborating databases, a substantial portion or all of the updated data can be transmitted or linked to the other collaborating database. Further, the transmitted data can include contextual metadata determined through surgical situational awareness and other additional or associated data, for example. Alternatively, some departments (e.g., billing) only need a small portion of certain data types. Accordingly, the data fluidity rules can dictate that when an update is made to a particular data type (or a set of data types) in a database, only a small portion of the updated data that is relevant to the recipient database is transmitted or linked to the recipient database. For example, if the recipient database is a billing department database, the data shared with the billing database may only include procedure codes, the time, and the expendables consumed during a medical procedure because only that data that is needed by the billing department. As can be seen, only data that is relevant to the recipient database is actually transmitted or linked to the recipient database, which limits access to sensitive patient data, prevents the recipient from being overwhelmed with unneeded data, and minimizes required data transmission bandwidths, while still allowing all connected databases to be seamlessly updated in accordance with each other.

In one implementation, the first database 212022 can include a laboratory database, the second database 212024 can include an EHR database, and the third database 212026 can include a hospital administration database. In this implementation of a data fluidity paradigm, the laboratory database and the administration database can transmit 212028 data 212029 between each other, the laboratory database and the EHR database can transmit 212030 data 212031 between each other, and the laboratory database and the administration database can transmit 212032 data between each other as dictated by the particular data fluidity ruleset defining the relations between the various databases. For example, the laboratory database could automatically transmit 212030 data 212031 including completed lab results to the EHR database to associate the lab results with the corresponding patient, whereafter the lab results can be retrieved from the EHR database. As another example, the laboratory database could automatically transmit 212028 data 212029 including a list of tests performed and other details to the hospital administration database, which can then be utilized to update billing information, reorder test supplies as needed, and so on. Further, each of the connections between the various aforementioned databases can be bidirectional. For example, if a patient's EHR is updated in the EHR database to include additional test results performed outside the given medical facility, those test results can likewise be automatically transmitted to the laboratory database for consideration and evaluation by the laboratory staff.

In another implementation, a computer system and/or network of linked databases can be configured to automatically collect and compile surgical outcomes resulting from specific treatment regimes by connecting the databases of various departments via a data fluidity paradigm, allowing all of the data pertaining to a patient's treatment to be aggregated and seamlessly integrated together. By automatically compiling patient outcome data with patient treatment data, patient care can be tracked more accurately and improvements can be developed for treatment regimes, surgical procedures, and other patient care. In some aspects, by automatically sharing relevant data across departments in a specific format for that department, the data can be more easily communicated, which can in turn allow the data to be presented more easily to patients, at meetings, in clinical papers, and so on. In some aspects, data can be recorded in each database and transmitted to the other connected databases in a standard format, allowing data from any given database to be seamlessly integrated into another compliant database.

In one aspect, collaboration across multiple departments could be increased by allowing or causing the data collected in any given database to easily flow from one group of specialists to another. The data fluidity paradigm allows for data to easily flow between departments at a medical facility by establishing a standard set of rules that all computer systems within the medical facility utilize to transmit or link data that dictates the destinations for any given type of data, the format that the data is to be transmitted in to the recipient database, and so on. The structured data-sharing paradigms described herein are beneficial in this and other contexts because they ensure that the correct data is being collected for physicians' uses. By allowing a computer system to automatically retrieve the necessary data from the relevant database(s) and having the databases update in concert with each other when data is added or changed, human errors in transmitting and transcribing data, errors due to receiving partial incomplete information, and other such errors are avoided.

In one aspect, some or all of the data in particular databases can respond fluidity to requests from users, rather than being automatically transmitted or linked to another database. Accordingly, a first computer system can be programmed to receive data requests from a second computer or database system (which can be initiated by a user, for example) and then transmit the requested data and/or define a relation between the database stored by the first computer system and the second computer system depending upon the identity or the type of request sent by the second computer system. For example, physicians can make data requests from the computer system, which then proceeds to automatically collect and compile the requested data from the relevant databases that the computer system is linked to. Such aspects can be utilized in a variety of applications, such as personalized cancer medicine. For example, the computer system can link the oncologist, surgeon, and histologist collaborating to treat a patient by allowing any of them to retrieve all of the treatment data related to the given patient. This in turn allows the medical personnel to each track the patient's treatment and allows the individual associated with a patient's care to easily retrieve and analyze data regarding the patient, such as a tumor location, margins, nodal dissection, and chemo treatment. By giving each individual associated with the treatment of a patient total access to the patient's data, follow-up and post-surgical treatment can be improved by ensuring that the medical personnel are all fully up to date on the patient's treatment. In some aspects, in addition to defining what information they would like to receive, the computer system can also be programmed to allow users to define the format that they would like the data to be presented in. Accordingly, the computer system can retrieve the identified data from the corresponding databases, convert the data to the desired format, and then present the data to the user.

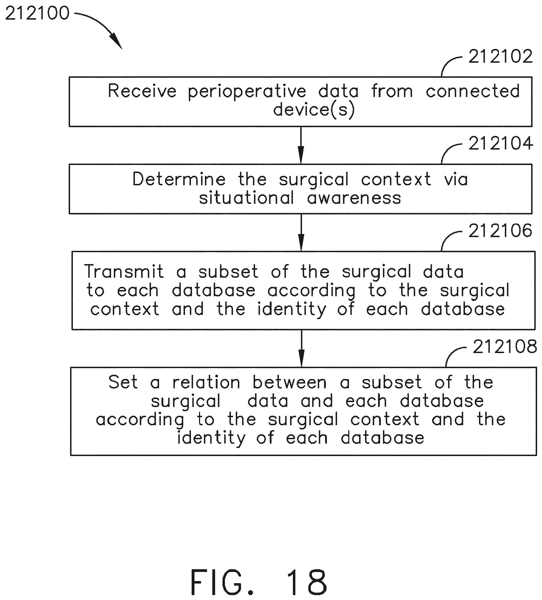

FIG. 18 illustrates one example of a process 212100 according to the structured data-sharing paradigms discussed herein where data is shared according to the surgical context associated with the data. As described above under the heading SURGICAL HUBS, computer systems, such as surgical hubs 106, 206 (FIGS. 1-11), can be connected to or paired with a variety of surgical devices, such as surgical instruments, generators, smoke evacuators, displays, and so on. Through their connections to these surgical devices, the surgical hubs 106, 206 can receive an array of perioperative data from these paired surgical devices while the devices are in use during a surgical procedure. Further, as described above under the heading SITUATIONAL AWARENESS, surgical hubs 106, 206 can determine the context of the surgical procedure being performed (e.g., the procedure type or the step of the procedure being performed) based, at least in part, on perioperative data received from these connected surgical devices. The surgical context determined by the surgical hub 106, 206 through situational awareness can be utilized to dictate what types of collected data are transmitted to particular databases, the format that the collected data is transmitted in, and so on. Accordingly, FIG. 18 is a logic flow diagram of a process 212100 for sharing data between databases, in accordance with at least one aspect of the present disclosure. The process 212100 can be executed by a processor or control circuit of a computer system, such as the processor 244 of the surgical hub 206 illustrated in FIG. 10. Accordingly, the process 212100 can be embodied as a set of computer-executable instructions stored in a memory 249 that, when executed by the processor 244, cause the computer system (e.g., a surgical hub 206) to perform the described steps.

Accordingly, the processor 244 executing the process 212100 receives 212102 perioperative data from the connected surgical devices and determines 212104 the surgical context based at least in part on the received perioperative data, as discussed above under the heading SITUATIONAL AWARENESS.

What the surgical hub 206 does with the collected data is dictated by the structured data ruleset being implemented by the surgical hub 206. Depending upon the surgical context and the type of data, the surgical hub 206 can transmit the data (or a subset thereof) to another database, set a relation between the database stored in the memory 249 of the surgical hub 206 and another database (i.e., link the relevant data fields of the databases), or take other such actions. In the illustrated aspect, the processor 244 transmits 212106 at least a portion of the collected surgical data to one or more recipient databases based on the determined surgical context and the identities of the recipient databases. The surgical data can include, for example, perioperative data received from the surgical devices, surgical contextual data determined via situational awareness (e.g., the surgery type or the step of the surgical procedure being performed), metadata associated with the surgical devices and/or the surgical context, and so on. Further, the processor 244 sets 212108 a relation between at least a portion of the collected surgical data stored in the surgical hub memory 249 and one or more recipient databases based the determined surgical context and the identities of the recipient databases. In other words, the surgical hub 206 transmits 212106 data and/or sets 212108 relations between its database and other databases according to the structured data-sharing ruleset, which defines which databases are to receive certain types of data or be linked to certain types of data collected by the surgical hub 206 based on the determined surgical context. For example, the surgical hub 206 could determine that a number of nonreusable surgical devices were used during the surgical procedure via situational awareness and accordingly transmit 212106 data indicating the types and numbers of nonreusable devices that were used to a purchasing database communicably connected to the surgical hub 206 for reordering of those nonreusable devices. The structured data-sharing ruleset can thus define that the purchasing database receives data related to consumed nonreusable surgical devices and that data is to be transmitted to the purchasing database. As another example, the surgical hub 206 could determine that the surgical procedure is completed or will be completed soon and accordingly set 212108 a relation between the data in its database storing the patient's biographical information and the surgical procedure type and a recovery department database to notify the recovery staff to prepare to receive the patient. The structured data-sharing ruleset can thus define that the recovery department database receives data related to identifying a patient and the surgery type and that data is to be linked to the recovery department database.

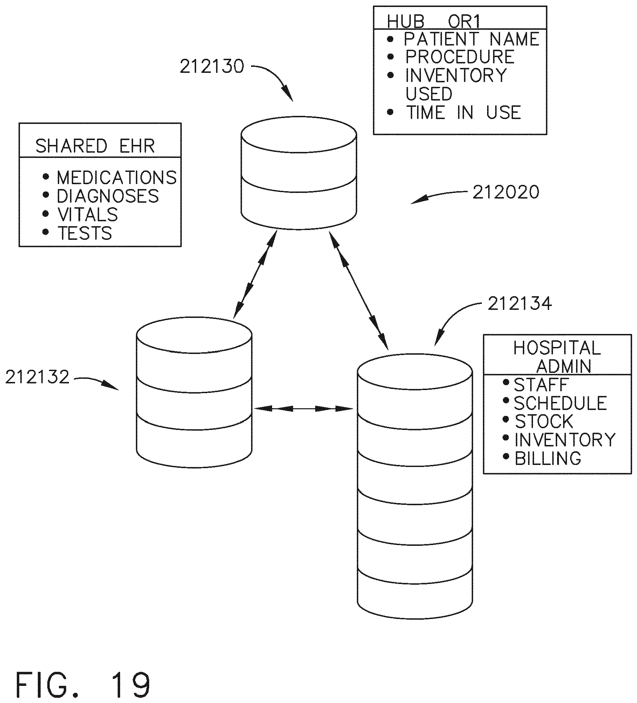

Another illustrative implementation of the process 212100 is depicted in FIG. 19. FIG. 19 is a diagram of a database system 212020 where particular data is shared between a surgical hub database 212130, an EHR database 212132, and a hospital administration database 212134, in accordance with at least one aspect of the present disclosure. The surgical hub database 212130 can collect a variety of data generated by the surgical hub 206 and/or any surgical devices paired with the surgical hub 206. For example, the surgical hub database 212130 can store the patient's name (or other biographical or identifying information), the surgical procedure undergone by the patient, the inventory of surgical devices and other products utilized during the surgical procedure, and/or the length of the surgical procedure. Further, the EHR database 212132 can store medications, diagnoses, vitals, and tests associated with the patient. Still further, the hospital administration database 212134 can store data including the hospital staff, scheduling, medical supply stock, inventory, and billing information. Each of the computer systems can be executing the process 212100 and, accordingly, can transmit the data stored in its respective database or set relations between their databases and the other databases as dictated by the particular data-sharing ruleset governing the interactions between each of the databases 212130, 212132, 212134.

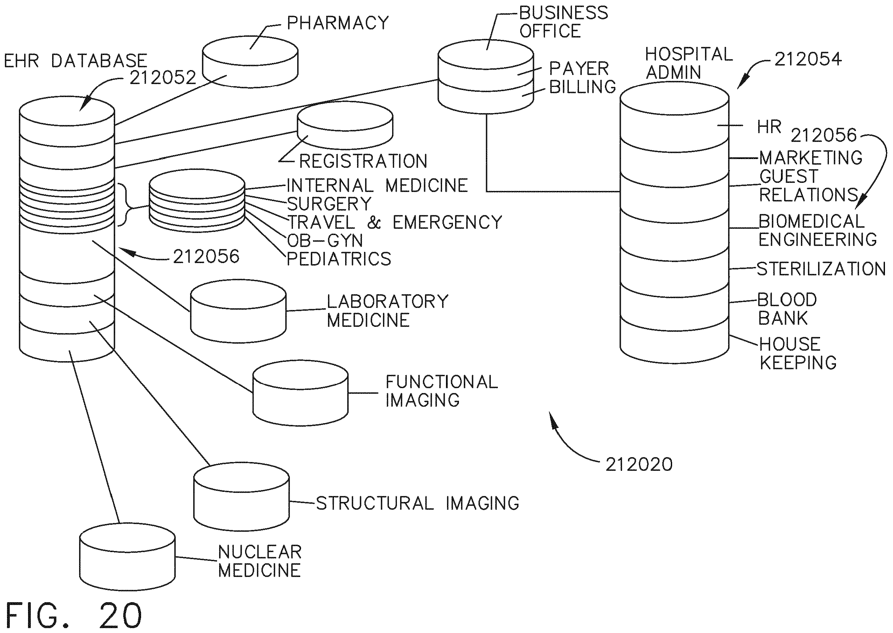

As discussed above, databases may only share a subset of the data they store with other connected databases. Further, different subsets of the data stored by each database may be shared with different databases, depending upon the data needed by the recipient databases. For example, data stored within each database can be organized into data categories and the structured data-sharing ruleset can dictate, for example, which data categories are shared with which other databases. For example, FIG. 20 depicts several illustrative data categories 212056 that the EHR database 212052 and the hospital administration database 212054 of the database system 212020 can store. In the depicted implementation, the business office data category, which includes payer and billing data as subcategories, is shared with (i.e., transmitted to or linked with) the hospital administration database 212054.The other data categories 212056 of the EHR database 212052 and the hospital administration database 212054 are not shared with the other database or are shared with other databases, as defined by the particular structured data-sharing ruleset.

The computer systems storing the databases 212130, 212132, 212134 that define a database system 212020 can be communicably linked together via, for example, a network. In some aspects, the computer systems can be cloud computing systems, as described above under the heading CLOUD SYSTEM HARDWARE AND FUNCTIONAL MODULES. In some aspects, multiple databases can be stored by a single computer system. In some aspects, the computer systems can be connected via a distributed computing communication protocol.

In one aspect, users can also define the types of data that they would like the medical facility's computer systems, such as the surgical hubs 106, 206 (FIGS. 1-11), to collect via, for example, a user interface provided by a computer system in the medical facility's network. For example, a user could indicate that they want the surgical hubs 206 in the medical facility to collect a particular type of data for a certain type of surgical instrument. Accordingly, the request can be pushed to the surgical hubs 206 within the medical facility network, and the surgical hubs 206 will thereafter collect that type of surgical instrument, if they are not already doing so. The surgical hubs 206 can collect intraoperative or postoperative data, as requested by the user. Once the request has been entered, the collected data can be shared with, for example, a database defined by the user according to a structured data-sharing ruleset, as described above. Thereafter, the data desired by the user can be transmitted, linked, or otherwise provided to the user. These aspects could be utilized to perform research on surgical instrument performance, correlations between patient outcomes and surgical techniques, and so on. In some aspects, the requested data can be forwarded to other users within or external to the medical facility network. In some aspects, the data request can be saved and repeated as desired by the user. In some aspects, the data request can proceed for a predefined period of time or indefinitely (until ended by the user). In some aspects, the user can follow up on the requested data by retrieving the metadata associated with the requested data or otherwise request other data that is associated with the requested data. For example, a user can enter a request to be provided with surgical device success rates. Accordingly, each surgical hub 206 or other computer system can monitor progress of each surgical procedure and device success rates associated therewith. Further, the user can cause the surgical hub 206 or other computer systems to route the surgical device success rate data to be transmitted to the re-ordering department (e.g., so that they know not to reorder surgical devices that have poor success rates) and any other desired department.

In various aspects, database systems executing a structured data-sharing paradigm can monitor the activities occurring in an OR through a surgical hub 206 therein and automatically route relevant data to relevant departments in order to improve the efficiency and function of the medical facility. In one aspect, a surgical hub 206 can be configured to monitor the progress of a surgical procedure, surgical device success rate, and other OR data via, for example, situational awareness. The ability of the surgical hub 206 to seamlessly share and communicate data with other databases in the medical facility can have a substantial number of benefits. For example, the surgical hub 206 can automatically share data regarding surgical device utilization with the re-ordering department through structured data sharing so that they know, for example, not to reorder surgical devices that have poor success rates. As another example, the surgical hub 206 can automatically share data regarding surgical outcomes with the pharmacy department so that they know, for example, that the patient may require additional pain medication due to a prolonged surgical procedure. As yet another example, the surgical hub 206 can automatically share data regarding any biopsies taken during the surgical procedure or other tissue samples that require testing with the pathology department so that they know, for example, to prepare to receive the tissue. As yet another example, the surgical hub 206 can automatically share data regarding the depletion of fluids (e.g., blood) during a surgical procedure with the medical supplies department so that they know to an order for backup supplies as the OR supply is depleted. As yet another example, the surgical hub 206 can automatically share data regarding an impending procedure with the medical supplies department so that they know, for example, to ready OR-specific drugs, hemostatic agents, and healing impacting agents (e.g., matrix metalloproteinase inhibitors) before the procedure. With the supplies readied ahead of time, they could then be delivered to the OR in a timely manner, allowing the surgical procedure to proceed on time and with the supplies at the correct usage temperature. Usage temperature can be important for certain types of agents, such as fibrin and thrombin. Fibrin and thrombin are refrigerated, biologically active agents that have to be dispensed at room temperature. If the surgical procedure calls for an agent, it can accordingly be critical for the adjunct to be at the correct temperature for the procedure. Through structured data sharing, a scheduling database can share scheduled surgical procedure times with all other relevant databases in the medical facility, ensuring that all relevant departments are fully up to date as to the start time for each procedure. If an agent is needed at the beginning of the procedure, then the medical facility personnel can be provided the precise time that the surgical procedure is to begin and can thus know to deliver the agent at that time. If an agent is needed during a procedure, a surgical hub 206 executing a situational awareness system can further monitor the progress of the surgical procedure after it has begun and update other relevant databases as to the status of the surgical procedure through structured data sharing so that medical facility personnel know the precise time at which they should bring desired agents to the OR so that they are maintained at the proper usage temperature. Accordingly, structured data sharing in the OR context can ensure that the agents are ready at the correct time, at the correct temperature, without risking any damage to the agents. As yet another example, the surgical hub 206 could monitor the progress of the surgical procedure (e.g., via situational awareness) and automatically share the procedural progress with the cleaning department so that they know when to expect to turn over the OR for the next procedure, which in turn aids in overall hospital logistics and scheduling by facilitating the process of cleaning and preparing surgical facilities for subsequent procedures.

In one aspect, a computer system (e.g., a surgical hub 206) can be programmed to track the use of surgical devices and their movement through a medical facility to, for example, collect data on the surgical instruments throughout their life cycle. Such data can include the number of times that a surgical device has been sterilized, repaired, and/or held in inventory or the amount of time that a surgical device has been held in each of the respective departments. A computer system can track surgical devices in this manner through structured data sharing by receiving from the databases of each relevant department location data for a surgical device (e.g., when a surgical device is brought to a department, it can be scanned into that department, which generates a record of the location of the surgical device), repair and maintenance records for the surgical device, and so on. Such data can be utilized to evaluate values, costs, and efficiencies of all of the medical products that are utilized in the medical facility.

In one aspect, a computer system can be programmed to allow patients to contribute self-reported data. In various aspects, the self-reported data could be directly entered into a database of a medical facility computer system via a computer terminal or the patient could cause a personal electronic device (or another personal data collection device) to automatically transmit collected information to a designated recipient database. The self-reported data could include, for example, blood sugar logs from testing equipment, such as a continuous blood glucose monitor, insulin pumps, artificial pancreas data, and so on. The self-reported data can also include, for example, data from activity monitors (e.g., Fitbit or Apple Watch) that are configured to collect activity data, location data, and other types of data. The activity monitors can provide, for example, activity level data (e.g., distance traveled, active minutes, number of steps taken, number of flights of stairs traversed), sleep data (e.g., sleep cycles, duration, and stages), heart rate monitoring data (e.g., resting heart rate, percent of time in specified heart rate zones, which can be determined by age, and heart rate variability), nutritional information, water intake, calories burned, and so on. When uploaded to a recipient database, the recipient database can then, in some aspects, automatically share relevant self-reported patient data with other connected devices according to a structured data-sharing ruleset.

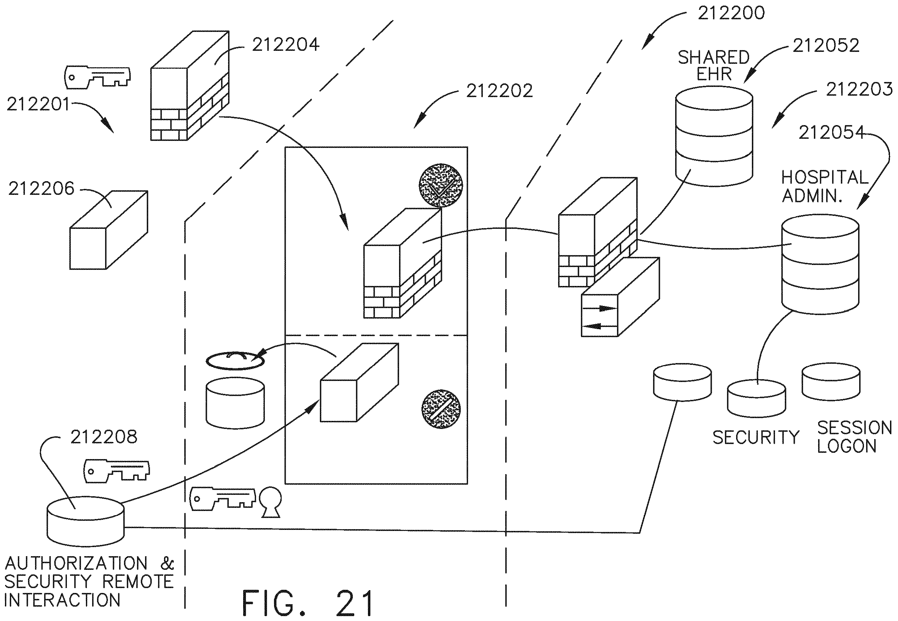

With structured data sharing, one concern is for access to data to only be granted to appropriate recipients. Accordingly, all data requests and all requests to link databases must be verified and authorized to prevent unauthorized recipients from gaining access to the data. FIG. 21 is a diagram illustrating a security and authorization system 212200 for a medical facility computer network 212203, in accordance with at least one aspect of the present disclosure. The security and authorization system 212200 can include, for example, a firewall 212202 to regulate incoming and outgoing data communication, such as communication requests 212201 from a computer system seeking to connect to the medical facility computer network 212203. Communication requests 212201 can include, for example, requests for particular data or data types to be transmitted from the medical facility computer network 212203, requests to establish a relation or link between a database in the medical facility computer network 212203 and an external database, and so on. In one aspect, communication requests 212201 can require a security key to be granted access to the medical facility computer network 212203. In one implementation, when the medical facility computer network 212203 receives a communication request 212201, the firewall 212202 can only permit access to the medical facility computer network 212203 if the security key corresponds to a valid security stored in an authorization database 212208, for example. Accordingly, authorized requests 212204 that have a valid security key will be granted access to the medical facility computer network 212203 and unauthorized requests 212206 lacking a valid security key will be denied access by the firewall 212202.

Accordingly, the structured data-sharing paradigms described herein, i.e., data fluidity and data interoperability, can facilitate the movement of data throughout a medical facility (or a network of interconnected medical facilities). By seamlessly sharing data so that every interconnected database always has access to all of the data generated in the medical facility that is relevant to its department, structured data-sharing paradigms allow medical facilities to operate more efficiently and provide better patient outcomes.

While several forms have been illustrated and described, it is not the intention of Applicant to restrict or limit the scope of the appended claims to such detail. Numerous modifications, variations, changes, substitutions and combinations to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms. The appended claims are intended to cover all such modifications, variations, changes, substitutions, modifications, and equivalents.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A computer system (206) configured to be communicably coupled to a surgical device and a database system, the computer system comprising:
a processor (244); and
a memory (249) coupled to the processor (244), the memory storing instructions that, when executed by the processor (244), cause the computer system to:
receive perioperative data from the surgical device;
determine a surgical context based at least in part on the perioperative data, the surgical context corresponding to surgical contextual data, wherein the surgical contextual data is selected from the group consisting of a procedure type, a procedure step, and a combination thereof;
define a relation between a first database stored in the memory (249) and a second database in the database system, the relation corresponding to the surgical context and an identity of the second database;
select a first subset of surgical data based on a property of the surgical data, wherein the surgical data comprises at least a portion of the perioperative data or the surgical contextual data, wherein the first subset corresponds to the surgical context and the identity of the second database, wherein the property corresponds to the surgical context, and wherein the property is selected from the group consisting of a bit size, a quantity, a resolution, a time bracket and any combination thereof; and
transmit the first subset of surgical data from the first database to the second database of the database system for storage thereon.

2. A computer-implemented method for sharing data between a computer system (206) and a database system, the computer system configured to be communicably coupled to a surgical device, the method comprising:
receiving, by the computer system (206), perioperative data from the surgical device;
determining, by the computer system (206), a surgical context based at least in part on the perioperative data, the surgical context corresponding to surgical contextual data; wherein the surgical contextual data is selected from the group consisting of a procedure type, a procedure step, and a combination thereof
defining, by the computer system (206), a relation between a first database stored in a memory (249) of the computer system and a second database in the database system, the relation corresponding to the surgical context and the identity of the second database;
selecting, by the computer system (206) a first subset of surgical data based on a property of the surgical data, wherein the surgical data comprises at least a portion of the perioperative data or the surgical contextual data, wherein the first subset corresponds to the surgical context and the identity of each of the one or more database, wherein the property corresponds to the surgical context, and wherein the property is selected from the group consisting of a bit size, a quantity, a resolution, a time bracket and any combination thereof;
transmitting, by the computer system (206), the first subset of the surgical data from the first database to the second database in the database system for storage thereon.

3. The computer system (206) of claim 1 or the computer-implemented method of claim 2, wherein the perioperative data comprises metadata associated with the surgical device.

4. The system or method of any preceding claim, wherein the computer system transmits the first subset of the surgical data and defines the relation between the database stored in the memory (249) and the second database without requiring action by a user.

5. The system or method of any preceding claim, wherein the identity of each of the plurality of databases correspond to departments of a medical facility.

6. A computer system (206) configured to be communicably coupled to a plurality of surgical devices and a database, the computer system comprising:
a processor (244); and
a memory (249) coupled to the processor (244), the memory storing instructions that, when executed by the processor, cause the computer system to:
receive perioperative data from the plurality of surgical devices;
determine a surgical context based at least in part on the perioperative data, the surgical context corresponding to surgical contextual data, wherein the surgical contextual data is selected from the group consisting of a procedure type, a procedure step, and a combination thereof;
receive a request for surgical data from the database;
select a first subset of surgical data based on a property of the surgical data, wherein the surgical data comprises at least a portion of the perioperative data or the surgical contextual data, wherein the property corresponds to the surgical context, and wherein the property is selected from the group consisting of a bit size, a quantity, a resolution, a time bracket and any combination thereof;
transmit the surgical data to the database according to an identity of the database; and
define a relation between the surgical data stored in the memory (249) and the database according to the identity of the database.

7. The computer system of claim 6, wherein the memory (249) stores instructions that, when executed by the processor (244), cause the computer system to:
receive a security key in association with the request;
authenticate the security key;
transmit the surgical data to the database according to whether the security key is authentic; and
define a relation between the surgical data stored in the memory (249) and the database according to the security key is authentic.

8. The computer system of claim 6 or claim 7, wherein the perioperative data comprises metadata associated with the surgical device.

9. The computer system of any one of claims 6 to 8, wherein the identity of the database corresponds to a department of a medical facility.

## Patentansprüche

1. Computersystem (206), das dafür ausgelegt ist, kommunikativ mit einer chirurgischen Vorrichtung und einem Datenbanksystem gekoppelt zu sein, wobei das Computersystem folgende Elemente umfasst:
einen Prozessor (244); und
einen Speicher (249), der mit dem Prozessor (244) gekoppelt ist, wobei der Speicher Anweisungen speichert, die bei ihrer Ausführung durch den Prozessor (244) das Computersystem zu folgenden Vorgängen veranlassen:
Empfangen von perioperativen Daten von der chirurgischen Vorrichtung;
Bestimmen eines chirurgischen Kontextes zumindest teilweise auf der Grundlage der perioperativen Daten, wobei der chirurgische Kontext chirurgischen Kontextdaten entspricht, wobei die chirurgischen Kontextdaten aus der Gruppe bestehend aus einer Verfahrensart, einem Verfahrensschritt und einer Kombination davon ausgewählt sind;
Definieren einer Beziehung zwischen einer ersten im Speicher (249) gespeicherten Datenbank und einer zweiten Datenbank im Datenbanksystem, wobei die Beziehung dem chirurgischen Kontext und einer Identität der zweiten Datenbank entspricht;
Auswählen einer ersten Teilmenge von chirurgischen Daten auf der Grundlage einer Eigenschaft der chirurgischen Daten, wobei die chirurgischen Daten mindestens einen Teil der perioperativen Daten oder der chirurgischen Kontextdaten umfassen, wobei die erste Teilmenge dem chirurgischen Kontext und der Identität der zweiten Datenbank entspricht, wobei die Eigenschaft dem chirurgischen Kontext entspricht und wobei die Eigenschaft aus der Gruppe bestehend aus einer Bitgröße, einer Menge, einer Auflösung, einem Zeitabschnitt und einer beliebigen Kombination davon ausgewählt ist; und
Übertragen der ersten Teilmenge chirurgischer Daten aus der ersten Datenbank an die zweite Datenbank des Datenbanksystems zur Speicherung in dieser.

2. Computerimplementiertes Verfahren zur gemeinsamen Nutzung von Daten zwischen einem Computersystem (206) und einem Datenbanksystem, wobei das Computersystem dafür ausgelegt ist, kommunikativ mit einer chirurgischen Vorrichtung gekoppelt zu sein, wobei das Verfahren folgende Vorgänge umfasst:
Empfangen, durch das Computersystem (206), von perioperativen Daten von der chirurgischen Vorrichtung;
Bestimmen, durch das Computersystem (206), eines chirurgischen Kontextes zumindest teilweise auf der Grundlage der perioperativen Daten, wobei der chirurgische Kontext chirurgischen Kontextdaten entspricht; wobei die chirurgischen Kontextdaten aus der Gruppe bestehend aus einer Verfahrensart, einem Verfahrensschritt und einer Kombination davon ausgewählt sind;
Definieren, durch das Computersystem (206), einer Beziehung zwischen einer ersten in einem Speicher (249) des Computersystems gespeicherten Datenbank und einer zweiten Datenbank im Datenbanksystem, wobei die Beziehung dem chirurgischen Kontext und der Identität der zweiten Datenbank entspricht;
Auswählen, durch das Computersystem (206), einer ersten Teilmenge von chirurgischen Daten auf der Grundlage einer Eigenschaft der chirurgischen Daten, wobei die chirurgischen Daten mindestens einen Teil der perioperativen Daten oder der chirurgischen Kontextdaten umfassen, wobei die erste Teilmenge dem chirurgischen Kontext und der Identität jeder von der einen oder den mehreren Datenbanken entspricht, wobei die Eigenschaft dem chirurgischen Kontext entspricht und wobei die Eigenschaft aus der Gruppe bestehend aus einer Bitgröße, einer Menge, einer Auflösung, einem Zeitabschnitt und einer beliebigen Kombination davon ausgewählt ist;
Übertragen, durch das Computersystem (206), der ersten Teilmenge der chirurgischen Daten aus der ersten Datenbank an die zweite Datenbank im Datenbanksystem zur Speicherung in dieser.

3. Computersystem (206) nach Anspruch 1 oder computerimplementiertes Verfahren nach Anspruch 2, wobei die perioperativen Daten Metadaten umfassen, die der chirurgischen Vorrichtung zugeordnet sind.

4. System oder Verfahren nach einem der vorhergehenden Ansprüche, wobei das Computersystem die erste Teilmenge der chirurgischen Daten überträgt und die Beziehung zwischen der im Speicher (249) gespeicherten Datenbank und der zweiten Datenbank definiert, ohne dass eine Aktion durch einen Benutzer erforderlich ist.

5. System oder Verfahren nach einem der vorhergehenden Ansprüche, wobei die Identität jeder der Vielzahl von Datenbanken Abteilungen einer medizinischen Einrichtung entspricht.

6. Computersystem (206), das dafür ausgelegt ist, kommunikativ mit einer Vielzahl von chirurgischen Vorrichtungen und einer Datenbank gekoppelt zu sein, wobei das Computersystem folgende Elemente umfasst:
einen Prozessor (244); und
einen Speicher (249), der mit dem Prozessor (244) gekoppelt ist, wobei der Speicher Anweisungen speichert, die bei ihrer Ausführung durch den Prozessor das Computersystem zu folgenden Vorgängen veranlassen:
Empfangen von perioperativen Daten von der Vielzahl von chirurgischen Vorrichtungen;
Bestimmen eines chirurgischen Kontextes zumindest teilweise auf der Grundlage der perioperativen Daten, wobei der chirurgische Kontext chirurgischen Kontextdaten entspricht, wobei die chirurgischen Kontextdaten aus der Gruppe bestehend aus einer Verfahrensart, einem Verfahrensschritt und einer Kombination davon ausgewählt sind;
Empfangen einer Anforderung nach chirurgischen Daten von der Datenbank;
Auswählen einer ersten Teilmenge von chirurgischen Daten auf der Grundlage einer Eigenschaft der chirurgischen Daten, wobei die chirurgischen Daten mindestens einen Teil der perioperativen Daten oder der chirurgischen Kontextdaten umfassen, wobei die Eigenschaft dem chirurgischen Kontext entspricht und wobei die Eigenschaft aus der Gruppe bestehend aus einer Bitgröße, einer Menge, einer Auflösung, einem Zeitabschnitt und einer beliebigen Kombination davon ausgewählt ist;
Übertragen der chirurgischen Daten an die Datenbank gemäß einer Identität der Datenbank; und
Definieren einer Beziehung zwischen den im Speicher (249) gespeicherten chirurgischen Daten und der Datenbank gemäß der Identität der Datenbank.

7. Computersystem nach Anspruch 6, wobei der Speicher (249) Anweisungen speichert, die bei ihrer Ausführung durch den Prozessor (244) das Computersystem zu folgenden Vorgängen veranlassen:
Empfangen eines Sicherheitsschlüssel im Zusammenhang mit der Anforderung;
Authentifizieren des Sicherheitsschlüssels;
Übertragen der chirurgischen Daten an die Datenbank in Abhängigkeit davon, ob der Sicherheitsschlüssel authentisch ist; und
Definieren einer Beziehung zwischen den im Speicher (249) gespeicherten chirurgischen Daten und der Datenbank in Abhängigkeit davon, ob der Sicherheitsschlüssel authentisch ist.

8. Computersystem nach Anspruch 6 oder Anspruch 7, wobei die perioperativen Daten Metadaten umfassen, die der chirurgischen Vorrichtung zugeordnet sind.

9. Computersystem nach einem der Ansprüche 6 bis 8, wobei die Identität der Datenbank einer Abteilung einer medizinischen Einrichtung entspricht.

## Revendications

1. Système informatique (206) configuré pour être couplé en communication à un dispositif chirurgical et à un système de bases de données, le système informatique comprenant :
un processeur (244) ; et
une mémoire (249) couplée au processeur (244), la mémoire stockant des instructions qui, lorsqu'elles sont exécutées par le processeur (244), forcent le système informatique à :
recevoir des données péri-opératoires en provenance du dispositif chirurgical ;
déterminer un contexte chirurgical sur la base au moins en partie des données péri-opératoires, le contexte chirurgical correspondant à des données contextuelles chirurgicales, dans lequel les données contextuelles chirurgicales sont sélectionnées parmi le groupe constitué par un type de procédure, une étape de procédure et une combinaison afférente ;
définir une relation entre une première base de données stockée dans la mémoire (249) et une seconde base de données dans le système de bases de données, la relation correspondant au contexte chirurgical et à une identité de la seconde base de données ;
sélectionner un premier sous-ensemble de données chirurgicales sur la base d'une propriété des données chirurgicales, dans lequel les données chirurgicales comprennent au moins une partie des données péri-opératoires ou des données contextuelles chirurgicales, dans lequel le premier sous-ensemble correspond au contexte chirurgical et à l'identité de la seconde base de données, dans lequel la propriété correspond au contexte chirurgical et dans lequel la propriété est sélectionnée parmi le groupe constitué par une dimension en nombre de bits, une quantité, une résolution, une parenthèse temporelle et une quelconque combinaison afférente ; et
transmettre le premier sous-ensemble de données chirurgicales depuis la première base de données sur la seconde base de données du système de bases de données pour un stockage dessus.

2. Procédé mis en œuvre par ordinateur pour partager des données entre un système informatique (206) et un système de bases de données, le système informatique étant configuré pour être couplé en communication à un dispositif chirurgical, le procédé comprenant :
la réception, par le système informatique (206), de données péri-opératoires en provenance du dispositif chirurgical ;
la détermination, par le système informatique (206), d'un contexte chirurgical sur la base au moins en partie des données péri-opératoires, le contexte chirurgical correspondant à des données contextuelles chirurgicales ; dans lequel les données contextuelles chirurgicales sont sélectionnées parmi le groupe constitué par un type de procédure, une étape de procédure et une combinaison afférente ;
la définition, par le système informatique (206), d'une relation entre une première base de données stockée dans une mémoire (249) du système informatique et une seconde base de données dans le système de bases de données, la relation correspondant au contexte chirurgical et à l'identité de la seconde base de données ;
la sélection, par le système informatique (206), d'un premier sous-ensemble de données chirurgicales sur la base d'une propriété des données chirurgicales, dans lequel les données chirurgicales comprennent au moins une partie des données péri-opératoires ou des données contextuelles chirurgicales, dans lequel le premier sous-ensemble correspond au contexte chirurgical et à l'identité de chacune des une ou plusieurs bases de données, dans lequel la propriété correspond au contexte chirurgical et dans lequel la propriété est sélectionnée parmi le groupe constitué par une dimension en nombre de bits, une quantité, une résolution, une parenthèse temporelle et une quelconque combinaison afférente ; et
la transmission, par le système informatique (206), du premier sous-ensemble de données chirurgicales depuis la première base de données sur la seconde base de données du système de bases de données pour un stockage dessus.

3. Système informatique (206) selon la revendication 1 ou procédé mis en œuvre par ordinateur selon la revendication 2, dans lequel les données péri-opératoires comprennent des métadonnées associées au dispositif chirurgical.

4. Système ou procédé selon l'une quelconque des revendications précédentes, dans lequel le système informatique transmet le premier sous-ensemble des données chirurgicales et définit la relation entre la base de données stockée dans la mémoire (249) et la seconde base de données sans la nécessité d'une action par un utilisateur.

5. Système ou procédé selon l'une quelconque des revendications précédentes, dans lequel les identités de chacune de la pluralité de bases de données correspondent à des départements d'un établissement médical.

6. Système informatique (206) configuré pour être couplé en communication à une pluralité de dispositifs chirurgicaux et à une base de données, le système informatique comprenant :
un processeur (244) ; et
une mémoire (249) couplée au processeur (244), la mémoire stockant des instructions qui, lorsqu'elles sont exécutées par le processeur, forcent le système informatique à :
recevoir des données péri-opératoires en provenance de la pluralité de dispositifs chirurgicaux ;
déterminer un contexte chirurgical sur la base au moins en partie des données péri-opératoires, le contexte chirurgical correspondant à des données contextuelles chirurgicales, dans lequel les données contextuelles chirurgicales sont sélectionnées parmi le groupe constitué par un type de procédure, une étape de procédure et une combinaison afférente ;
recevoir une requête demandant des données chirurgicales à partir de la base de données ;
sélectionner un premier sous-ensemble de données chirurgicales sur la base d'une propriété des données chirurgicales, dans lequel les données chirurgicales comprennent au moins une partie des données péri-opératoires ou des données contextuelles chirurgicales, dans lequel la propriété correspond au contexte chirurgical et dans lequel la propriété est sélectionnée parmi le groupe constitué par une dimension en nombre de bits, une quantité, une résolution, une parenthèse temporelle et une quelconque combinaison afférente ;
transmettre les données chirurgicales à la base de données conformément à une identité de la base de données ; et
définir une relation entre les données chirurgicales stockées dans la mémoire (249) et la base de données conformément à l'identité de la base de données.

7. Système informatique selon la revendication 6, dans lequel la mémoire (249) stocke des instructions qui, lorsqu'elles sont exécutées par le processeur (244), forcent le système informatique à :
recevoir une clé de sécurité en association avec la requête ;
authentifier la clé de sécurité ;
transmettre les données chirurgicales à la base de données en fonction de si la clé de sécurité est authentique ; et
définir une relation entre les données chirurgicales stockées dans la mémoire (249) et la base de données en fonction du fait que la clé de sécurité est authentique.

8. Système informatique selon la revendication 6 ou la revendication 7, dans lequel les données péri-opératoires comprennent des métadonnées associées au dispositif chirurgical.

9. Système informatique selon l'une quelconque des revendications 6 à 8, dans lequel l'identité de la base de données correspond à un département d'un établissement médical.
